(19) Europäisches Patentamt
European Patent Office
Office européen des brevets

(11) EP 4 755 914 A1

(12) **EUROPEAN PATENT APPLICATION**
published in accordance with Art. 153(4) EPC

(43) Date of publication:
**10.06.2026 Bulletin 2026/24**

(21) Application number: **24848296.0**

(22) Date of filing: **31.07.2024**

(51) International Patent Classification (IPC):
***C07K 16/28*** *(2006.01)* ***A61K 47/68*** *(2017.01)*
***A61P 35/00*** *(2006.01)*

(52) Cooperative Patent Classification (CPC):
**A61K 47/68; A61P 35/00; C07K 16/28**

(86) International application number:
**PCT/CN2024/108742**

(87) International publication number:
**WO 2025/026341 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.07.2023 CN 202310944619**

(71) Applicant: **CSPC Megalith Biopharmaceutical Co., Ltd.**
**Shijiazhuang, Hebei 050025 (CN)**

(72) Inventors:
- **SHEN, Mingyue**
  **Shijiazhuang, Hebei 050025 (CN)**
- **WANG, Yancui**
  **Shijiazhuang, Hebei 050025 (CN)**
- **SUN, Zhaopeng**
  **Shijiazhuang, Hebei 050025 (CN)**
- **YUAN, Can**
  **Shijiazhuang, Hebei 050025 (CN)**
- **WEN, Penghui**
  **Shijiazhuang, Hebei 050025 (CN)**
- **HUI, Xiwu**
  **Shijiazhuang, Hebei 050025 (CN)**
- **DAN, Mo**
  **Shijiazhuang, Hebei 050025 (CN)**
- **YAO, Bing**
  **Shijiazhuang, Hebei 050025 (CN)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

# (54) ANTI-ROR1 PROTEIN ANTIBODY AND CONJUGATE THEREOF

(57) An anti-ROR1 antibody, an antibody-drug conjugate containing the antibody, a pharmaceutical composition and medical product containing the antibody-drug conjugate, and a method for preparing the antibody-drug conjugate, and the use thereof. The antibody-drug conjugate can be effectively used for treating and/or preventing ROR1 positive tumors including cancers represented by hematologic neoplasms and solid tumors, such as breast cancer and bladder cancer.

## Description

### TECHNICAL FIELD

The present application pertains to the field of biomedicine, and more particularly relates to an anti-ROR1 protein antibody, a conjugate comprising the antibody and use of the antibody or conjugate in the treatment or prevention of tumors.

### BACKGROUND

Receptor-tyrosine-kinase-like orphan receptor 1, known as ROR1, is a type I single transmembrane protein, which is a member of the receptor tyrosine kinase (RTK) family. ROR1 is expressed at a low level during embryonic development but at a high level in various malignant tumors or tissues, such as chronic lymphocytic leukemia (CLL), polar lymphocytic leukemia (ALL), breast cancer, ovarian cancer, melanoma, and lung adenocarcinoma. It is shown in extensive data that ROR1 plays a significant role in promoting tumor growth and metastasis, inducing tumor cells' resistance to drugs, and inhibiting apoptosis. Human ROR1 molecule consists of an extracellular region, a transmembrane region, and an intracellular region. The extracellular region contains an immunoglobulin-like domain (Ig-like), a cysteine-rich or frizzled domain (CRD or FZD), and a Kring (KNG) domain (KRD). The intracellular region contains a tyrosine kinase domain (TKD), two serine/threonine-rich domains (Ser/ThrD), and a proline-rich domain (PRD).

Studies have shown that the known physiological functions of ROR1 include (1) playing an important role in mouse growth which is characterized in that ROR1 is expressed in the face, limbs, heart and lungs of mice and although ROR1-deficient mice can survive, they exhibit respiratory disorders and die within 24 hours of birth; and (2) playing a key role in regulating the development of embryonic muscles and bones during embryonic development, including regulating cell division, proliferation and migration, and being involved in the formation of organs such as nerves, bones and blood vessels.

Overexpression of ROR1 can promote tumor growth during cancer progression. ROR1 can bind to Wnt5a, be involved in Wnt signal transduction, and interact with signaling pathways such as EGFR and Met, and promote the growth, proliferation, and migration of tumor cells. ROR1 and CK1ε (casein kinase 1) can activate AKT/PI3K, thereby activating the pCREB pathway and leading to cell proliferation. ROR1 can enhance EGFR signaling and induce epithelial-mesenchymal transition (EMT). ROR1 is highly expressed in most hematologic malignancies and solid tumors. For example, the proportion of ROR1+ cells exceeds 90% in CLL patients. In some solid tumors, such as lung cancer, pancreatic cancer, and colorectal cancer cells, ROR1 expression is also significantly increased and closely related to disease progression and treatment efficacy. Therefore, ROR1 can serve as a specific targeted biomarker for the development of cancer therapeutic drugs.

Breast cancer is still a female tumor with the highest incidence in the world, and the global incidence rate of breast cancer is increased by 0.2% -8% every year. About 1.4 million people are diagnosed with breast cancer every year, and approximately 500,000 people are die from breast cancer, which is the leading cause of death for females aged 40-55 years. Since the late 1970s, breast cancer has consistently been the leading malignant tumors in females worldwide. According to a report from the American Cancer Society, there are approximately 200,000 new cases of breast cancer in America each year, with an incidence rate of 116 per 100,000. For American females, the life risk of developing breast cancer by age 85 is about 12.5%, meaning that 1 in 8-10 females will develop breast cancer, while the life risk of dying from breast cancer is about 3.4%. However, with the development of medical conditions and the continuous research and development of new drugs, although the global incidence of breast cancer has increased by 3% per year over the past one decade, the survival rate of breast cancer patients has increased by 20%. In China, where the incidence rate of breast cancer is low as compared with other countries, the rate has significantly increased in recent years. Over the past one decade, the incidence of breast cancer in the major cities in China has increased by 37%, and the mortality rate has increased by 38.9%, while the mortality rate in rural areas in China has increased by 39.7%. Alarmingly, the annual incidence rate increase in breast cancer in China is higher than that in countries with high incidence rates by 1-2 percentage, increasing at a rate of 3-4% each year. Breast cancer has become the cancer with the most mortality rate increase in cities, and the age of onset is also showing a trend of becoming younger. Shanghai, Beijing, Tianjin, and coastal areas are particularly high-incidence areas for breast cancer in China, where breast cancer has become the leading cause of malignant tumors in females.

Endometrial cancer, also known as uterine cancer, is one of the three most common malignant tumors of the female reproductive tract, and it mostly occurs in perimenopausal and postmenopausal women. With the increase in average life expectancy and changes in lifestyle, the incidence of endometrial cancer has been rising and becoming more prevalent among the younger over the past two decades. In western countries, endometrial cancer has become the leading malignant tumors of the female reproductive system. In China, according to the Epidemic Situation Analysis of Malignant Tumors in China in 2015 published by the National Cancer Center in 2019, there were approximately 69,000

cases of endometrial cancer in 2015, with 16,000 deaths, an incidence rate of 10.28 per 100,000, accounting for 3.88% of morbidity of female malignant tumor. As the second most common gynecological malignancy following cervical cancer, endometrial cancer accounts for approximately 20% to 30% of gynecological malignancies. In some developed cities, the incidence of endometrial cancer has become the highest among gynecological malignancies.

Prostate cancer is the most common cancer in males, accounting for 13% of all cancer cases. Prostate cancer-related death accounts for 7% of all cancer-related deaths, making it the second leading cause of cancer death in males. Over 41 years, the age-standardized incidence of prostate cancer has increased by 1,993%, although the mortality rate has decreased by 10% over the past decade. Prostate cancer is a heterogeneous disease with a wide range of pathogenesis, from asymptomatic and long-term cases to fatal cases of the disease or severe malignant tumors with a significant incidence rate.

Currently, among drugs targeting ROR1, there are four ADC drugs, one humanized monoclonal antibody drug, five bispecific antibody drugs, and four CAR-T drugs, with Phase III being most advanced clinical stage. No product has been approved for marketing. Among those ADC drugs currently in clinical trials, VLS101 is in Phase III clinical trials. In October 2021, VelosBio reported Phase I clinical data for VLS101 in the treatment of lymphoma, showing good clinical results. However, due to nonspecific conjugation, the small-molecule payload shedding caused by retro-Michael addition leads to blood toxicity, raising safety concerns. There is a need in the market for more therapeutic drugs targeting ROR1 to meet the diverse treatment requirements of patients.

## SUMMARY OF THE INVENTION

In a first aspect, the present application provides a conjugate comprising an anti-ROR1 antibody or an antigen-binding fragment thereof conjugated to one or more drug molecules.

In a second aspect, the present application provides a pharmaceutical composition comprising the conjugate described in the first aspect and a pharmaceutically acceptable carrier.

In a third aspect, the present application provides use of the conjugate described in the first aspect or the pharmaceutical composition described in the second aspect in the manufacture of a medicament for the treatment or prevention of cancer.

In a fourth aspect, the present application provides a method for treating cancer in a subject, comprising administering to the subject suffering from the cancer a therapeutically effective amount of the conjugate described in the first aspect or the pharmaceutical composition described in the second aspect.

In a fifth aspect, the present application provides a medical preparation (e.g., a kit) comprising the conjugate described in the first aspect or the pharmaceutical composition described in the second aspect.

In a sixth aspect, the present application provides use of the conjugate described in the first aspect and an antiproliferative agent in the manufacture of a medicament for the treatment or prevention of tumors.

In a seventh aspect, the present application provides a pharmaceutical composition comprising the conjugate described in the first aspect and an antiproliferative agent.

In an eighth aspect, the present application provides a method for treating a tumor in a subject, comprising administering to a subject suffering from the tumor a therapeutically effective amount of the conjugate described in the first aspect or the pharmaceutical composition described in the second aspect together with an antiproliferative agent

In a ninth aspect, the present application provides an anti-ROR1 antibody or an antigen-binding fragment thereof, a pharmaceutical composition comprising the antibody or antigen-binding fragment, pharmaceutical use of the antibody or antigen-binding fragment, and a method for treating a tumor/cancer using the antibody or antigen-binding fragment.

In one embodiment, the anti-ROR1 antibody or antigen-binding fragment thereof of the present application comprises a heavy chain variable region and a light chain variable region, wherein:

1) the heavy chain variable region comprises CDR1, CDR2 and CDR3 having the sequences as set forth in SEQ ID NOs: 1, 2 and 3, respectively, and the light chain variable region comprises CDR1, CDR2 and CDR3 having the sequences as set forth in SEQ ID NOs: 4, 5 and 6, respectively; or
2) the heavy chain variable region comprises CDR1, CDR2 and CDR3 having the sequences as set forth in SEQ ID NOs: 1, 9 and 3, respectively, and the light chain variable region comprises CDR1, CDR2 and CDR3 having the sequences as set forth in SEQ ID NOs: 4, 5 and 6, respectively; or
3) the heavy chain variable region comprises CDR1, CDR2 and CDR3 having the sequences as set forth in SEQ ID NOs: 1, 11 and 3, respectively, and the light chain variable region comprises CDR1, CDR2 and CDR3 having the sequences as set forth in SEQ ID NOs: 4, 5 and 6, respectively.

In some embodiments, the antibody can be a monoclonal antibody, a bispecific antibody, or a humanized antibody.

In some embodiments, the antibody can be an IgG-type antibody, preferably an IgG1-type antibody.

In some embodiments, the antigen-binding fragment can be a Fab fragment, an F(ab')$_2$ fragment, or a single-chain Fv fragment (scFv).

In some embodiments, the heavy chain variable region has the amino acid sequence as set forth in SEQ ID NO: 7, 10 or 12, and/or the light chain variable region has the amino acid sequence as set forth in SEQ ID NO: 8.

In some embodiments, the antibody can comprise a heavy chain comprising a heavy chain constant region having the amino acid sequence as set forth in SEQ ID NO: 13, and/or the antibody can comprise a light chain comprising a light chain constant region having the amino acid sequence as set forth in SEQ ID NO: 19.

In some embodiments, the antibody or antigen-binding fragment comprise a light chain having a C-terminus attached to a glutamine-containing peptide tag, and the glutamine-containing peptide tag is selected from the group consisting of LQSGA, GGLQSGA and GGGLQSGA.

In another aspect, the present application provides a conjugate comprising the anti-ROR1 antibody or antigen-binding fragment thereof as described in any one of the above embodiments conjugated to one or more drug molecules, wherein the conjugate has a structure represented by Ab-(L-U)n, where Ab represents the anti-ROR1 antibody or antigen-binding fragment thereof, L represents a linker, U represents the drug molecule, and n is an integer or decimal within the range of 1 to 8, such as 1, 2, 3, 4, 5, 6, 7, or 8, preferably 4.

In some embodiments, the drug molecule can be an anticancer drug, such as a cytotoxic drug, an immunopotentiator, or a radioisotope.

In some embodiments, the cytotoxic drug can be selected from the group consisting of a tubulin inhibitor, a DNA topoisomerase inhibitor, a DNA damaging agent, an anti-metabolite, and an anti-tumor antibiotic.

In some embodiments, the tubulin inhibitor can be selected from the group consisting of an auristatin derivative (e.g., MMAE (Monomethyl auristatin E) or MMAF (Monomethyl auristatin F)), and a maytansine alkaloid derivative (e.g., DM1, DM4, Ansamitocin, Mertansine, or Dolastatin or a derivative thereof).

In some embodiments, the DNA topoisomerase inhibitor can be selected from the group consisting of a camptothecin analogue, and a DNA topoisomerase I inhibitor or a derivative thereof, for example, DXD, SN38, irinotecan, irinotecan hydrochloride, camptothecin, 9-aminocamptothecin, 9-nitrocamptothecin, 10-hydroxycamptothecin, 9-chloro-10-hydroxycamptothecin, 22-hydroxyacuminatine, topotecan, lertonotecan, belotecan, ixitecan, homosilatecan, 6,8-dibromo-2-methyl-3-[2-(D-xylopyranosylamino)phenyl]-4(3H)-quinazolinone, 2-cyano-3-(3,4-dihydroxyphenyl)-N-(phenylmethyl)-(2E)-2-acrylamide, 2-cyano-3-(3,4-dihydroxyphenyl)-N-(3-hydroxyphenylpropyl)-(E)-2-acrylamide, 12-β-D-glucopyranosyl-12,13-dihydro-2,10-dihydroxy-6-[[2-hydroxy-1-(hydroxymethyl)ethyl]amino]-5H-indoleindolo[2,3-a]pyrrolo[3,4-c]carbazole-5,7(6H)-dione, N-[2-(dimethylamino)ethyl]-4-acridine formamide dihydrochloride, or N-[2-(dimethylamino)ethyl]-4-acridinecarboxamide.

In some embodiments, the DNA damaging agent can be selected from the group consisting of calicheamicin, duocarmycin, and an anthramycin derivative PBD (pyrrolobenzodiazepine).

In some embodiments, the anti-metabolite can be selected from the group consisting of methotrexate, 6-mercaptopurine, and 5-fluorouracil.

In some embodiments, the anti-tumor antibiotic can be selected from the group consisting of a polypeptide antibiotic (e.g., actinomycin D or bleomycin) and an anthraquinone (e.g., doxorubicin or mitoxantrone hydrochloride).

In some embodiments, the immunopotentiator can be selected from the group consisting of levamisole, pidotimod, imiquimod, isoprinosine, polyinosinic:polycytidylic acid, and polyinosinic:polyuridinic acid.

In some embodiments, the radioisotope can be selected from the group consisting of $^{211}$At, $^{131}$I, $^{125}$I, $^{90}$Y, $^{186}$Re, $^{188}$Re, $^{153}$Sm, $^{212}$Bi, $^{32}$P, $^{60}$Co, and $^{177}$Lu.

In some embodiments, the drug molecule can be conjugated to the antibody or antigen-binding fragment via a linker, and the linker can be linked to the antibody or antigen-binding fragment via a sulfhydryl or an amino group.

In some embodiments, the linker can be independently selected from the group consisting of mc-Val-Cit-pAB, mc-Val-Cit-pABC, mc-Val-Cit, $NH_2$-$(PEG)_m$-Val-Cit, $NH_2$-$(PEG)_m$-Val-Cit-pAB, and $NH_2$-$(PEG)_m$-Val-Cit-pABC, where the m in $(PEG)_m$ is an integer within the range of 1 to 8, preferably 3.

In some embodiments, the antibody or antigen-binding fragment can comprise a light chain having a C-terminus attached to a glutamine-containing peptide tag selected from the group consisting of LQSGA, GGLQSGA and GGGLQSGA, and the linker is linked to the side chain of the glutamine residue from the glutamine-containing peptide tag via an amide bond.

In some embodiments, the antibody can comprise a heavy chain constant region comprising Q295, and the linker is linked to the side chain of Q295 via an amide bond.

In some embodiments, the linker is linked to the side chain of the glutamine residue from the glutamine-containing peptide tag via an amide bond.

In yet another aspect, the present application further provides a pharmaceutical composition, comprising the conjugate as described in any one of the above embodiments, a pharmaceutically acceptable carrier, and optionally, an antiproliferative agent.

In some embodiments, the antiproliferative agent can be selected from the group consisting of paclitaxel,

doxorubicin, docetaxel, cisplatin, carboplatin, and iproplatin.

In yet another aspect, the present application further provides use of the conjugate or the pharmaceutical composition as described in any one of the above embodiments in the manufacture of a medicament for the treatment or prevention of cancer.

In yet another aspect, the present application further provides a method for treating cancer in a subject, comprising administering to the subject suffering from the cancer a therapeutically effective amount of the conjugate or the pharmaceutical composition as described in any one of the above embodiments.

In some embodiments, the cancer is a solid tumor, such as gastric cancer, esophageal cancer, pancreatic cancer, lung cancer, ovarian cancer, colon cancer, liver cancer, head and neck cancer, or gallbladder cancer; preferably, an adenocarcinoma of stomach, esophagus, pancreatic duct, bile duct, lung, or ovary; more preferably, gastric cancer or pancreatic cancer.

In some embodiments, the cancer is a hematologic tumor, preferably mantle cell lymphoma, chronic lymphocytic leukemia, diffuse large B-cell lymphoma, follicular lymphoma, or Richter-transformed lymphoma.

In some embodiments, the cancer is ROR1-positive cancer.

In yet another aspect, the present application further provides a medical preparation, comprising the conjugate or the pharmaceutical composition as described in any one of the above embodiments. The medical preparation can be a kit comprising a container, containing the conjugate or the pharmaceutical composition.

## BRIEF DESCRIPTION OF THE DRAWINGS

FIG. 1 shows the illustrated structure of the conjugate Hu17-H2L1/H3L1/H4L1-4LND1002 ADC according to an exemplary embodiment of the present application. The left part shows the antibody structure, and the right part shows the corresponding linker-drug structure. Each molecule consists of one anti-ROR1 humanized monoclonal antibody (Hu17-H2L1/H3L1/H4L1) with one molecule of MMAE or a derivative thereof conjugated to Q295 (EU numbering scheme) of each heavy chain, and a glutamine-containing peptide tag (GGLQSGA) attached to the C-terminus (carboxyl terminus) of each light chain. The peptide tag is conjugated to one MMAE derivative molecule via a linker. The antibody and linker are linked via a stable amide bond (isopeptide bond). The average drug-to-antibody ratio (DAR) is 4.0. The right part shows the structure of LND1002 before conjugation, including a drug molecule such as MMAE or a derivative thereof, and a linker $NH_2$-$PEG_3$-Val-Cit-pABC. After conjugation, the terminal $NH_2$ group of the linker is linked to Q295 of the heavy chain or the side chain of the glutamine residue (Q) from the glutamine-containing peptide tag at the terminus of the light chain ($-CH_2-CH_2-C(=O)-NH_2$) to form "$-CH_2-CH_2-C(=O)-NH-PEG_3$-Val-Cit-pABC" structure.

FIG. 2 shows the binding affinities of murine and humanized antibodies to MDA-MB-231-ROR1 at cellular levels.

FIG. 3 shows detection spectrum of UC961-VCMMAE DAR.

FIG. 4 shows endocytosis results of various ADCs in MDA-MB-231-ROR1 cells.

FIG. 5 shows the curves of inhibitory effects of various ADCs on human lung adenocarcinoma H1975 mouse xenograft models.

FIG. 6 shows the curves of inhibitory effects of various ADCs on human breast cancer HCC1187 mouse xenograft models.

FIG. 7 shows concentration-time curves of the two ADCs following three administrations to cynomolgus monkeys.

FIG. 8 shows concentration-time curves of free small molecule drugs following three administrations of two ADCs to cynomolgus monkeys.

## DETAILED DESCRIPTION OF THE INVENTION

### DEFINITIONS

Unless defined otherwise, all scientific and technical terms used herein have the same meaning as understood by one of ordinary skill in the art. For definitions and terms in the art, a person skilled in the art may refer specifically to Current Protocols in Molecular Biology (Ausubel). Abbreviations for amino acid residues are standard 3-letter and/or 1-letter codes used in the art to refer to one of the 20 common L-amino acids.

Although the numerical ranges and parameter approximations are shown in broad ranges in the present application, the numerical values shown in the specific embodiments are described as accurately as possible. However, any numerical values inherently contain certain errors due to the standard deviation present in their respective measurements. Additionally, all ranges disclosed herein are to be understood as encompassing any and all subranges contained therein. For example, a range of "1 to 10" shall be considered to encompass any and all subranges between the

minimum value 1 and the maximum value 10, inclusive, i.e., all subranges starting with a lower limit of 1 or more, such as 1 to 6.1, and subranges ending with an upper limit of 10 or less, such as 5.5 to 10. Additionally, any reference referred to as "incorporated herein" is to be understood as being incorporated in its entirety.

As used herein, the terms "pharmaceutical composition", "combinational drug", and "pharmaceutical combination" can be used interchangeably to refer to a combination of at least one drug and, optionally, a pharmaceutically acceptable carrier or adjuvant that is combined together to achieve a particular purpose. In certain embodiments, a pharmaceutical composition includes a combination of components which are temporally and/or spatially separated, so long as the components are capable of acting together to achieve a purpose of the present application. For example, the ingredients contained in the pharmaceutical composition (e.g., antibodies, nucleic acid molecules, nucleic acid molecule combinations and/or conjugates according to the present application) may be administered to a subject together or separately. When ingredients contained in the pharmaceutical composition are separately administered to a subject, the ingredients may be administered to the subject simultaneously or sequentially. Preferably, a pharmaceutically acceptable carrier is water, a buffered aqueous solution, an isotonic salt solution such as PBS (phosphate buffer), glucose, mannitol, dextrose, lactose, starch, magnesium stearate, cellulose, magnesium carbonate, 0.3% glycerol, hyaluronic acid, ethanol, or polyalkylene glycols such as polypropylene glycol, or triglycerides. The type of a pharmaceutically acceptable carrier depends, inter alia, on whether the composition according to the present application is formulated for oral, nasal, intradermal, subcutaneous, intramuscular or intravenous administration. The compositions according to the present application may comprise wetting agents, emulsifying agents or buffer substances as additives.

The pharmaceutical compositions, vaccines or pharmaceutical formulations according to the present application may be administered by any suitable route, for example orally, nasally, intradermally, subcutaneously, intramuscularly or intravenously.

As used herein, "a therapeutically effective amount" or "an effective amount" refers to a dose sufficient to cause benefit to a subject who receives an administration. The actual administration dose, rate and course will depend on the condition and disease severity of the subject to be treated.

As used herein, the term "subject" refers to mammal, such as human, as well as other animals, such as wild animals (e.g., herons, storks, or cranes), domestic animals (e.g., ducks, or geese), or experimental animals (e.g., gorillas, monkeys, rats, mice, rabbits, guinea pigs, woodchucks, or ground squirrels).

In broad sense, the term "antibody" encompasses intact antibodies and any antigen-binding fragment ("antigen-binding portion") or single chain form thereof. "Full-length/intact antibody" refers to a protein comprising at least two heavy (H) chains and two light (L) chains interconnected by disulfide bonds. Each heavy chain contains a heavy chain variable region (VH) and a heavy chain constant region containing three domains CH1, CH2 and CH3. Each light chain contains a light chain variable region (VL) and a light chain constant region containing one domain CL. VH and VL regions can be further divided into a plurality of regions with high variabilities, referred to as complementarity determining regions (CDRs). Among CDRs, there are more conservative regions referred to as framework regions (FRs). Each VH or VL consists of three CDRs and four FRs arranged from the amino terminus to the carboxy terminus in the following order: FR1, CDR1, FR2, CDR2, FR3, CDR3, and FR4. These variable regions of heavy and light chains contain binding domains that interact with antigens. The constant region of an antibody can mediate binding of immunoglobulins to tissues or factors in hosts, including various cells of the immune system (e.g., effector cells) and the first component of the classical complement system (Clq). Chimeric or humanized antibodies are also encompassed by antibodies according to the present application. A full-length/intact antibody may be any type of antibodies, such as IgD, IgE, IgG, IgA or IgM antibodies (or subclasses of the above), but not necessarily belonging to any particular class. Immunoglobulins can be assigned to different classes depending on the amino acid sequences of the heavy chain constant domain of antibodies. Generally, immunoglobulins have five main classes, i.e., IgA, IgD, IgE, IgG and IgM. Several of these classes can be further classified into subclasses (isotypes), such as IgG1, IgG2, IgG3, IgG4, IgA1 and IgA2. Heavy chain constant domains corresponding to various classes of immunoglobulins are referred to as $\alpha$, $\delta$, $\varepsilon$, $\gamma$ and $\mu$, respectively. Subunit structures and three-dimensional structures of various classes of immunoglobulins are well known.

Complementarity determining regions (CDRs, typically including CDR1, CDR2 and CDR3) are the subregions in variable regions that most impact on the affinity and specificity of an antibody. CDR sequences within a VH or VL domain can be defined using many common identification systems, including the Kabat numbering scheme, the IMGT numbering scheme, the Chothia numbering system, and the Contact-based numbering system. For a variable region sequence of a given antibody, the CDR sequences within the VH and VL domains can be defined according to Kabat, IMGT, Chothia, or Contact numbering system. In the present application, unless defined otherwise, the amino acid sequences of CDRs described herein (e.g. SEQ ID Nos. 1-6, 9, and 11) are defined according to the Kabat numbering scheme. However, it is well known to in the art that the CDRs of an antibody can be defined with various methods. Although the protection scopes of claims of the present application are based on the sequences identified according to the Kabat numbering scheme, corresponding amino acid sequences from other CDR definition systems should also fall within the protection scope of the present application.

The term "humanized antibody" refers to an antibody that may comprise a CDR region derived from a human

antibody, and other portions derived from one (or several) human antibodies. Moreover, in order to retain binding affinity, some residues of the backbone segments (referred to as FR) can be modified. Humanized antibodies or fragments thereof according to the present application can be prepared by any technique known to those skilled in the art.

The term "semi-humanized antibody" is defined in relation to a humanized antibody or a fully humanized antibody, and refers to an antibody which has one chain comprising a murine variable region (as in a chimeric antibody) and the other chain comprising a humanized variable region.

The term "chimeric antibody" refers to an antibody in which the variable region sequence is from one species and the constant region sequence is from another species, e.g., an antibody in which the variable region sequence is from a mouse antibody and the constant region sequence is from a human antibody. Chimeric antibodies or fragments thereof according to the present application can be prepared by using genetic recombination techniques. For example, a chimeric antibody may be produced by cloning a recombinant DNA comprising a promoter and a sequence encoding a variable region of a non-human, particularly murine, monoclonal antibody according to the present application, and a sequence encoding a constant region of a human antibody. Chimeric antibodies of the present application encoded by such recombinant genes will be, for example, a murine-human chimera, and its specificity is determined by the variable regions derived from murine DNA and its isoforms are determined by the constant regions derived from human DNA. For a method of preparing a chimeric antibody, guidelines can be found in, for example, Verhoeyn et al. (BioEssays, 8: 74, 1988).

The term "monoclonal antibody" refers to a preparation of antibody molecules having same molecular compositions. Monoclonal antibody compositions exhibit a single binding specificity and affinity for a particular epitope.

The term "bispecific antibody" refers to an antibody having binding capacities for two antigenic epitopes. The two antigenic epitopes may be on different antigens or on a same antigen. Bispecific antibodies may have a variety of structural configurations. For example, a bispecific antibody may consist of two Fc fragments and two binding moieties fused thereto, respectively (similar to a native antibody, except that the two arms bind different antigen targets or epitopes). The antigen binding moiety may be a single chain antibody (scfv) or a Fab fragment.

As used herein, the term "antigen-binding fragment" refers in particular to antibody fragments such as Fv, scFv ("sc" denotes single chain), Fab, $F(ab')_2$, Fab', scFv-Fc fragment or diabody, or any fragment which is capable of increasing the half-life by chemical modification or by incorporation into liposomes, such as addition of poly(alkylene) glycols, such as polyethylene glycol ("pegylated") (referred to as pegylated fragments Fv-PEG, scFv-PEG, Fab-PEG, $F(ab')_2$-PEG or Fab'-PEG) ("PEG" represents polyethylene glycol). An antigen-binding fragment of an anti-ROR1 antibody of the present application has ROR1 binding activity. For example, an antigen-binding fragment consists of or comprises a partial sequence of the heavy or light chain variable region of the antibody from which it is derived. The partial sequence is sufficient to retain the same binding specificity and sufficient affinity as the antibody from which it is derived, and may comprise at least 5 amino acids, preferably 10, 15, 25, 50 and 100 contiguous amino acids of the antibody sequence from which it is derived.

Examples of antigen-binding fragments include, but are not limited to, (1) Fab fragments, which may be monovalent fragments having VL-CL chains and VH-CH1 chains; (2) $F(ab')_2$ fragments, which may be divalent fragments having two Fab' fragments linked by disulfide bridges of the hinge region (i.e., dimers of Fab'); (3) Fv fragments having VL and VH domains from a single arm of an antibody; (4) single chain Fv (scFv), which may be a single polypeptide chain consisting of a VH domain and a VL domain linked by a peptide linker; and (5) $(scFv)_2$, which may comprise two VH domains linked by a peptide linker and two VL domains that are combined with the two VH domains via disulfide bridges.

The terms "Fc fragment", "Fc domain", "Fc moiety" or the like refer to a portion of the constant region of an antibody heavy chain, including the hinge region, the CH2 fragment and CH3 fragment of the constant region.

Generally, in order to prepare a monoclonal antibody or an antigen-binding fragment thereof, in particular a murine monoclonal antibody or an antigen-binding fragment thereof, guidelines can be found in the techniques described in "Antibodies" manual (Harlow and Lane, Antibodies: A Laboratory Manual, Cold Spring Harbor Laboratory, Cold Spring Harbor NY, pp.726, 1988) or techniques for preparing a monoclonal antibody from hybridoma cells described by Kohler and Milstein (Nature, 256: 495-497, 1975).

The term "homology/identity" in the context of an amino acid or nucleic acid sequence is defined as the percentage of identical residues in an amino acid or nucleotide sequence variant that, after alignment and introduction of gaps, achieves maximum percent homology, if desired. Methods and computer programs for alignment are well known in the art.

The term "specific binding" refers to a non-random binding reaction between two molecules, such as binding of an antibody to an antigenic epitope.

The term "ADC" refers to antibody-drug conjugate, in which an small molecule active drug is conjugated to an antibody or antigen-binding fragment thereof via a linker through chemical bonds. As used herein, the terms "ADC", "antibody drug conjugate", and "conjugate" can be used interchangeably.

As used herein, the term "cancer" refers to a proliferative disorder caused by or characterized by cell proliferation in which the cells have lost their susceptibility to normal growth control. The term "cancer" includes tumors and any other proliferative disorders. Cancers of a same tissue type originate from the same tissue and can be classified into different

subtypes based on their biological characteristics.

The term "UC961-vc-MMAE" is also known as VLS-101, which is an ADC prepared from the conjugation of the tubulin polymerization inhibitor MMAE to a cysteine residue of the antibody Cirmtuzumab (UC961) via the cleavable linker MC-Val-Cit-PAB, with average DAR value of 4. The antibody sequence can be found in sequence 3-6 of CN111587124A.

As used herein, the terms "$NH_2$-$PEG_3$-Val-Cit" and "$NH_2$-$PEG_3$-Val-Cit-pABC" have the same meaning, and refer to the following structure:

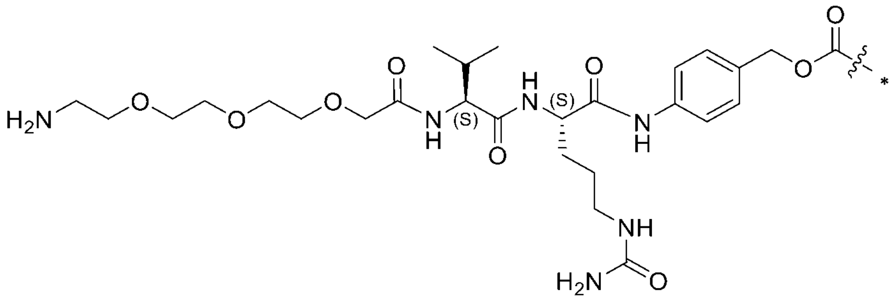

where * represents the position for linking to a bioactive molecule X, such as MMAE.

When linked to an antibody, it has the following structure:

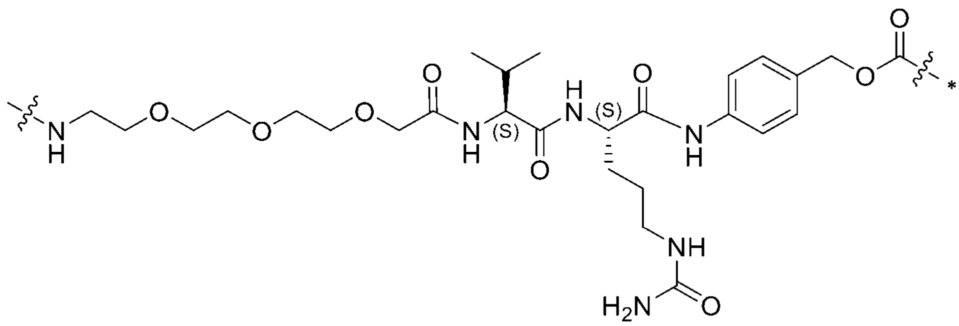

where the amino group (-NH-) is linked to an antibody.

The term "MMAE" refers to monomethyl auristatin E, and has the following structure:

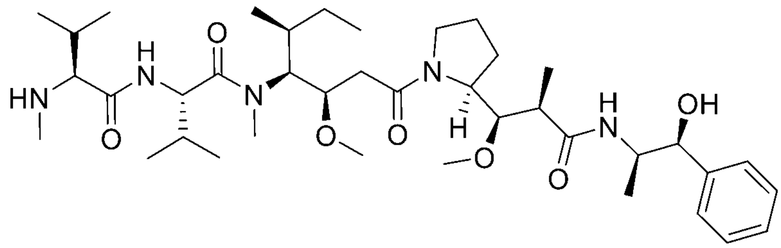

"MMAE" has the following structure when linked to a linker:

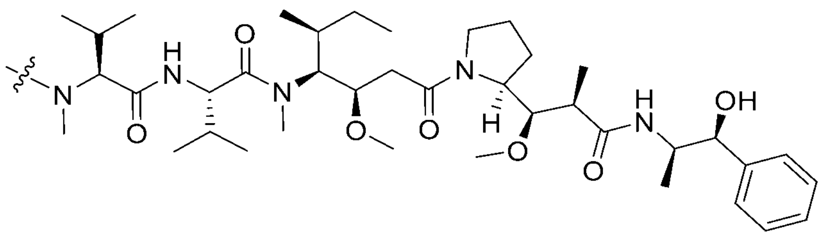

## The ROR1 antibodies and conjugates of the present application

To address the shortcomings in the prior art, it was developed in the present application a humanized anti-ROR1

antibody that can bind to ROR1 on ROR1-positive cells and be internalized efficiently, making it highly suitable for ADC development. A drug molecule, such as MMAE, can be conjugated to the anti-ROR1 antibody via mTgase enzyme to obtain an antibody-drug conjugate with DAR of 4 and excellent homogeneity. In subsequent ADC research and development, it was discovered that using the linker of the present application can achieve good stability in the conjugation between the linker and the drug molecule, thereby enabling the humanized antibody to be conjugated to a small molecule drug (e.g., MMAE) via the linker, resulting in an ADC drug that has strong killing effects on ROR1-overexpressing cancer cells, especially pancreatic cancer, gastric cancer, and lung cancer cells, and also has good stability. Furthermore, the ADCs of the present application have a unique design, which includes a glutamine-containing peptide segment GGLQSGA at the terminus of the light chain to achieve a higher drug loading with DAR=4, thereby improving the therapeutic efficacy of the drug without significantly increasing toxicity. It was shown in *in vivo* experiments that intravenous administration of the antibody-drug conjugate resulted in dose-dependent tumor growth inhibition in nude mice with ROR1-positive gastric or pancreatic tumor xenografts, demonstrating significant overall therapeutic effects. Moreover, the ADCs of the present application can produce a bystander effect, further enhancing the therapeutic effect. At the same dosage, the *in vivo* efficacy of the ADCs of the present application are superior to that of VLS101. In a preliminary toxicology study in cynomolgus monkeys, the ADCs of the present application administered at 9 mg/kg showed similar hematologic toxicity to that observed for VLS101 administered at 6 mg/kg, significantly improving the therapeutic window.

In one aspect, the present application provides an antibody or antigen-binding fragment thereof capable of specifically binding to ROR1. In particular, the antibody or antigen-binding fragment thereof comprises a heavy chain variable region and a light chain variable region, wherein (i) the heavy chain variable region comprises three CDR regions, and at least one of the CDR regions has the amino acid sequence as set forth in SEQ ID NO: 1, 2 or 3, or a sequence having at least 80% (preferably 85%, 90%, 95%, 98%, or 99%) sequence identity to the amino acid sequence as set forth in SEQ ID NO: 1, 2 or 3; and/or (ii) the light chain variable region comprises three CDR regions, and at least one of the CDR regions has the amino acid sequence as set forth in SEQ ID NO: 4, 5 or 6, or a sequence having at least 80% (preferably 85%, 90%, 95%, 98% or 99%) sequence identity to the amino acid sequence as set forth in SEQ ID NO: 4, 5 or 6.

In some embodiments, the antibody or antigen-binding fragment thereof comprises a heavy chain variable region and a light chain variable region, wherein (i) the heavy chain variable region comprises three CDR regions having the amino acid sequences as set forth in SEQ ID NOs: 1, 2 and 3, respectively; and/or (ii) the light chain variable region comprises three CDR regions having the amino acid sequences as set forth in SEQ ID NOs: 4, 5 and 6, respectively.

In some embodiments, the antibody or antigen-binding fragment thereof comprises a heavy chain variable region and a light chain variable region, wherein (i) the heavy chain variable region comprises three CDR regions having the amino acid sequences as set forth in SEQ ID NOs: 1, 9 and 3, respectively; and/or (ii) the light chain variable region comprises three CDR regions having the amino acid sequences as set forth in SEQ ID NOs: 4, 5 and 6, respectively.

In some embodiments, the antibody or antigen-binding fragment thereof comprises a heavy chain variable region and a light chain variable region, wherein (i) the heavy chain variable region comprises three CDR regions having the amino acid sequences as set forth in SEQ ID NOs: 1, 11 and 3, respectively; and/or (ii) the light chain variable region comprises three CDR regions having the amino acid sequences as set forth in SEQ ID NOs: 4, 5 and 6, respectively.

In some embodiments, the antibody or antigen-binding fragment thereof comprises a heavy chain variable region and a light chain variable region, wherein (i) the heavy chain variable region comprises three CDR regions having the amino acid sequences GFSLSTSGMG (SEQ ID NO: 20), IWWDDDK (SEQ ID NO: 21), and ARPQFITTVVAFYWYFDV (SEQ ID NO: 22), respectively; and/or (ii) the light chain variable region comprises three CDR regions having the amino acid sequences ENIYSN (SEQ ID NO: 23), AAT, and QHFWGTPWT (SEQ ID NO: 6), respectively. The CDRs are defined according to the IMGT numbering scheme.

In some embodiments, the antibody or antigen-binding fragment thereof comprises a heavy chain variable region and a light chain variable region, wherein (i) the heavy chain variable region comprises three CDR regions having the amino acid sequences GFSLSTSGM (SEQ ID NO: 24), WWDDD (SEQ ID NO: 25), and PQFITTVVAFYWYFDV (SEQ ID NO: 3), respectively; and/or (ii) the light chain variable region comprises three CDR regions having the amino acid sequences RASENIYSNLA (SEQ ID NO: 4), AATNLAD (SEQ ID NO: 5), and QHFWGTPWT (SEQ ID NO: 6), respectively. The CDRs are defined according to the Chothia numbering system.

In some embodiments, the antibody or antigen-binding fragment thereof comprises a heavy chain variable region and a light chain variable region, wherein (i) the heavy chain variable region comprises three CDR regions having the amino acid sequences STSGMGVA (SEQ ID NO: 26), WLAHIWWDDDKR (SEQ ID NO: 27), and ARPQFITTVVAFYWYFD (SEQ ID NO: 28), respectively; and/or (ii) the light chain variable region comprises three CDR regions having the amino acid sequences as set forth in YSNLAWY (SEQ ID NO: 29), LLVYAATNLA (SEQ ID NO: 30), and QHFWGTPW (SEQ ID NO: 31), respectively. The CDRs are defined according to the Contact-based numbering system.

In some embodiments, the antibody or antigen-binding fragment thereof of the present application is isolated.

In some embodiments, the heavy chain variable region has the amino acid sequence as set forth in SEQ ID NO: 7, and/or the light chain variable region has the amino acid sequence as set forth in SEQ ID NO: 8.

In some embodiments, the heavy chain variable region has the amino acid sequence as set forth in SEQ ID NO:

10, and/or the light chain variable region has the amino acid sequence as set forth in SEQ ID NO: 8.

In some embodiments, the heavy chain variable region has the amino acid sequence as set forth in SEQ ID NO: 12, and/or the light chain variable region has the amino acid sequence as set forth in SEQ ID NO: 8.

In some embodiments, the antibody of the present application is a monoclonal antibody.

In some embodiments, the antibody of the present application is a bispecific antibody. For example, one arm of the bispecific antibody can be the antigen-binding fragment (e.g., Fab or scfv) of the anti-ROR1 antibody of the present application, and the other arm can be an antigen-binding fragment (e.g., Fab or scfv) that targets another antigen (e.g., other antigen targets useful for ADC construction) or another ROR1 epitope (e.g., different from the ROR1 epitope to which the anti-ROR1 antibody of the present application binds).

In some embodiments, the antibody of the present application is a humanized antibody, including a semi-humanized antibody.

In some embodiments, the antibody or antigen-binding fragment thereof of the present application has ADCC activity.

In some embodiments, the antibody or an antigen-binding fragment thereof of the present application has CDC activity.

In some embodiments, the antibody comprises a heavy chain constant region of IgG1 subtype, IgG2 subtype, or IgG4 subtype.

In some embodiments, the antibody comprises a heavy chain constant region of human IgG1 subtype, human IgG2 subtype, human IgG4 subtype, murine IgG1 subtype, or murine IgG2a subtype.

In some embodiments, the antibody comprises a heavy chain constant region of IgG1 subtype. That is, the antibody is an IgG1-type antibody.

In some embodiments, the antibody comprises a light chain constant region of κ subtype or λ subtype.

In some embodiments, the antibody comprises a light chain constant region of human κ subtype, human λ subtype, murine κ subtype, or murine λ subtype.

In some embodiments, the antibody comprises a heavy chain constant region of human IgG1 subtype containing mutation(s) at one or more of positions L234, L235, P329, and P331 (according to the EU numbering scheme). For example, the mutation(s) is one or more of L234A, L235A, P329A, and P331S.

In some embodiments, the antibody comprises a heavy chain constant region of human IgG1 subtype, containing four mutations: L234A, L235A, P329A, and P331S.

In some embodiments, the antibody of the present application is an IgG1κ antibody.

In some embodiments, the antibody comprises a heavy chain comprising a heavy chain constant region having the amino acid sequence as set forth in SEQ ID NOs: 13, and/or a light chain comprising a light chain constant region having the amino acid sequence as set forth in SEQ ID NO: 19. In some embodiments, the antibody comprises a heavy chain comprising a heavy chain constant region having the amino acid sequence as set forth in SEQ ID NOs: 13, and/or a light chain comprising a light chain constant region having the amino acid sequence as set forth in SEQ ID NO: 14.

In some embodiments, the antibody or antigen-binding fragment thereof of the present application can be used to treat or prevent cancer which overexpresses ROR1.

In one embodiment, an antibody capable of binding to ROR1 binds to a natural epitope of ROR1 present on the surface of living cells. In one embodiment, an antibody capable of binding to ROR1 binds to the extracellular domain of ROR1. In one embodiment, an antibody capable of binding to ROR1 binds to the extracellular region of ROR1.

In some embodiments, the present application provides a full-length antibody comprising a glutamine-containing peptide tag attached to the C-terminus (carboxyl terminus) of a light chain of the antibody.

In some embodiments, the glutamine-containing peptide tag includes LQSGA, GGLQSGA, or GGGLQSGA.

In another aspect, the present application provides an isolated polynucleotide encoding the antibody of the present application.

In yet another aspect, the present application provides a combination of isolated polynucleotides comprising a polynucleotide encoding the light chain of the antibody or antigen-binding fragment thereof of the present application and a polynucleotide encoding the heavy chain of the antibody or antigen-binding fragment thereof of the present application.

In another aspect, the present application provides an expression vector comprising a polynucleotide as described herein or a combination of polynucleotides as described herein, and the polynucleotide(s) is operably linked to a regulatory sequence that allows expression of the polypeptide(s) encoded by the polynucleotide(s) in a host cell or cell-free expression system.

In some embodiments of the present application, the host cell can be a prokaryotic host cell, a eukaryotic host cell, or a phage. The prokaryotic host cell can be *Escherichia coli, Bacillus subtilis, Streptomyces,* or *Proteus mirabilis.* The eukaryotic host cell can be fungi, such as *Pichia pastoris, Saccharomyces cerevisiae, Schizosaccharomyces cerevisiae,* or *Trichoderma;* insect cells such as *Spodoptera frugiperda*; plant cells such as tobacco; or mammal cells, such as BHK cells, CHO cells, COS cells, or myeloma cells. In some embodiments, the host cells described herein are preferably mammal cells, more preferably BHK cells, CHO cells, NSO cells, or COS cells.

In another aspect, the present application provides an antibody-drug conjugate comprising an anti-ROR1 antibody or an antigen-binding fragment thereof of the present application conjugated to one or more drug molecules. The embodiments and features of the anti-ROR1 antibody or antigen-binding fragment thereof of the present application are described above.

Since ROR1 is primarily a molecular target for cancer/tumor cells, in some embodiments, the drug molecule is an anticancer drug. However, it should be appreciated by those skilled in the art that when ROR1 is chosen as a target for diseases other than cancer/tumor, the drug molecule can be selected according to the disease of interest.

Anticancer drugs include, but are not limited to, a cytotoxic drug, an immunopotentiator, or a radioisotope.

In some embodiments, the cytotoxic drug includes a tubulin inhibitor (e.g., an alkaloid), a DNA topoisomera se inhibitor, a DNA damaging agent, an antimetabolite, or an antitumor antibiotic.

In some embodiments, the cytotoxic drug can be selected from the group consisting of, for example, anti-tubulin agents, DNA alkylating agents, DNA cross-linking agents, DNA intercalating agents, and RNA polymerase II inhibitors. In some embodiments, the cytotoxic drug is selected from the group consisting of: monomethyl auristatin E (MMAE), azonafide, α-amanitin, Duocarmycin™, pyrrolobenzodiazepine (PBD), anthracycline neomycin metabolite PNU-159682 (CAS No: 202350-68-3), and pharmaceutically acceptable salts, esters, or analogs thereof.

In some embodiments, a tubulin inhibitor includes, but is not limited to, an auristatin derivative (e.g., MMAE (Monomethyl auristatin E), or MMAF (Monomethyl auristatin F)) or a maytansine alkaloid derivative (e.g., DM1, DM4, Ansamitocin, Mertansine, or dolastatin or a derivative thereof).

In some embodiments, a DNA topoisomerase inhibitor is a camptothecin analog, or a DNA topoisomerase I inhibitor or a derivative thereof, such as DXD, SN38, irinotecan, irinotecan hydrochloride, camptothecin, 9-aminocamptothecin, 9-nitrocamptothecin, 10-hydroxycamptothecin, 9-chloro-10-hydroxycamptothecin, 22-hydroxyacuminatine, topotecan, lertonotecan, belotecan, ixitecan, homosilatecan, 6,8-dibromo-2-methyl-3-[2-(D-xylopyranosylamino)phenyl]-4(3H)-quinazolinone, 2-cyano-3-(3,4-dihydroxyphenyl)-N-(phenylmethyl)-(2E)-2-acrylamide, 2-cyano-3-(3,4-dihydroxyphenyl)-N-(3-hydroxyphenylpropyl)-(E)-2-acrylamide, 12-β-D-glucopyranosyl-12,13-dihydro-2,10-dihydroxy-6-[[2-hydroxy-1-(hydroxymethyl)ethyl]amino]-5H-indoleindolo [2,3-a]pyrrolo[3,4-c]carbazole-5,7(6H)-dione, N-[2-(dimethylamino)ethyl]-4-acridine formamide dihydrochloride, or N-[2-(dimethylamino)ethyl]-4-acridinecarboxamide.

In some embodiments, a DNA damaging agent includes, but is not limited to, calicheamicin, duocarmycin, or pyrrolobenzodiazepine PBD (an anthramycin derivative).

In some embodiments, an immunopotentiator includes, but is not limited to, levamisole, pidomod, imiquimod, isoinosine, polyinosinic:polycytidylic acid, or polyinosinic:polyuridinic acid.

In some embodiments, an antimetabolite includes, but is not limited to, methotrexate, 6-mercaptopurine, or 5-fluorouracil.

In some embodiments, anti-tumor antibiotics include, but are not limited to, polypeptide antibiotics (e.g., actinomycin D or bleomycin) or anthraquinones (e.g., doxorubicin or mitoxantrone hydrochloride).

In some embodiments, an radioisotope includes, but is not limited to, $^{211}At$, $^{131}I$, $^{125}I$, $^{90}Y$, $^{186}Re$, $^{188}Re$, $^{153}Sm$, $^{212}Bi$, $^{32}P$, $^{60}Co$, or $^{177}Lu$.

In some embodiments, an antibody capable of binding to ROR1 is covalently linked to a drug moiety via a linker. In some embodiments, the linker is a cleavable linker. In some embodiments, the linker can be cleaved under intracellular conditions. In one embodiment, the linker can subject to hydrolysis at a pH lower than 5.5. In some embodiments, the linker can be cleaved by an intracellular protease. In some embodiments, the linker is a cathepsin cleavable linker. In some embodiments, the linker comprises a dipeptide. In some embodiments, the dipeptide is valine (Val)-citrulline (Cit). In some embodiments, the antibody is attached to the linker through a thiol group of a cysteine of the antibody. In one embodiment, the antibody is attached to the linker through an amino group of the antibody, in particular an amino group of a glutamine residue.

Non-limiting examples of the linker include mc-Val-Cit-pAB, mc-Val-Cit-pABC, mc-Val-Cit, $NH_2$-$(PEG)_m$-Val-Cit, $NH_2$-$(PEG)_m$-Val-Cit-pAB and $NH_2$-$(PEG)_m$-Val-Cit-pABC, where m is an integer within the range of 1 to 8.

In some embodiments, the antibody-drug conjugate of the present application has the general formula Ab-(L-U) n, where Ab represents the anti-ROR1 antibody of the present application, L is a linker (e.g., $NH_2$-$(PEG)_m$-Val-Cit, $NH_2$-$(PEG)_m$-Val-Cit-pAB, $NH_2$-$(PEG)_m$-Val-Cit-pABC, mc-Val-Cit-pAB, or Val-Cit, wherein m represents the number of PEG, which may be an integer within the range of 1 to 8), and U is a drug (e.g., DM1, DM4, MMAE, MMAF, DXD or SN38), and n represents a drug antibody ratio (DAR). The DAR value can be an average value, and can be any value within the range of 1 to 8 (not limited to an integer, but may also be a decimal), preferably an integer within the range of 1 to 8, more preferably 2, 4, 6 or 8, even more preferably 2.

In another aspect, the present application provides a pharmaceutical formulation (e.g., a pharmaceutical composition) comprising an antibody-drug conjugate of the present application, and a pharmaceutically acceptable diluent, carrier, or excipient.

In yet another aspect, the present application provides a medical preparation comprising an antibody-drug

conjugate of the present application. In some embodiments, the medical preparation is in the form of a kit comprising a container which contains the antibody-drug conjugate of the present application. In one embodiment, the medical preparation further comprises printed instructions for using the preparation in a method of treating or preventing cancer, in particular cancer expressing ROR1.

The antibody-drug conjugates provided herein are effective in treating and/or preventing cancer associated with cells expressing ROR1. By way of non-limiting example, the cancer can be gastric cancer, esophageal cancer, pancreatic cancer, lung cancer (e.g., non-small cell lung cancer (NSCLC)), ovarian cancer, colon cancer, liver cancer, head and neck cancer, or gallbladder cancer, or metastases of the aforementioned cancer, in particular gastric metastases, peritoneal metastases, or lymph node metastases. Cancer suitable for being treated with the antibody-drug conjugate provided herein can be adenocarcinomas of stomach, esophagus, pancreatic duct, bile duct, lung and ovary. The antibody-drug conjugates provided herein are particularly suitable for the treatment of gastric cancer, pancreatic cancer, breast cancer and bladder cancer.

In some embodiments, the cancer is chronic lymphocytic leukemia (CLL), T-cell leukemia (TCL), mantle cell lymphoma (MCL), diffuse large B-cell lymphoma (DLBCL), Burkitt's lymphoma, multiple myeloma (MM), marginal zone lymphoma (MZL), small lymphocytic lymphoma (SLL), or non-Hodgkin's lymphoma that has undergone Richter transformation (NHL).

In some embodiments, the cancer to be treated can also be selected from the group consisting of, for example, lymphoma, small lymphocytic lymphoma, marginal zone lymphoma, marginal cell B-cell lymphoma, Burkitt's lymphoma, mantle cell lymphoma, diffuse large B-cell lymphoma, non-Hodgkin's lymphoma, chronic lymphocytic leukemia, osteosarcoma, renal cell carcinoma, hepatocellular carcinoma, colon cancer, colorectal cancer, breast cancer, epithelial squamous cell carcinoma, melanoma, myeloma, multiple myeloma, brain cancer, non-small cell lung cancer, cervical cancer, ovarian cancer, liver cancer, prostate cancer, testicular cancer, thyroid cancer, and head and neck cancer.

Therefore, the present application also provides several inventions related to the above-mentioned therapeutic uses.

In one aspect, the present application provides the use of the antibody-drug conjugate described herein in the preparation of medicaments for the treatment or prevention of cancer.

In another aspect, the present application provides a method for treating cancer in a subject, comprising administering to the subject suffering from the cancer a therapeutically effective amount of the antibody-drug conjugate, or a pharmaceutical formulation or composition comprising the antibody-drug conjugate described herein.

In another aspect, the present application provides use of the antibody-drug conjugate described herein and an antiproliferative agent in the preparation of medicament for treating tumors (such as the cancers described herein).

In another aspect, the present application provides a pharmaceutical composition comprising the antibody-drug conjugate described herein and an antiproliferative agent.

In another aspect, the present application provides a method for treating a tumor in a subject, comprising administering to the subject suffering from the tumor a therapeutically effective amount of the antibody-drug conjugate, or a pharmaceutical formulation or composition comprising the antibody-drug conjugate as described herein and an antiproliferative agent.

In some embodiments, the antiproliferative agent includes, but is not limited to, paclitaxel, doxorubicin, docetaxel, cisplatin, carboplatin, and isopropylplatin.

In some embodiments, the antiproliferative agent can also be another antibody, antibody-drug conjugate, or fusion protein.

In yet another aspect, the present application provides suitable TGases, such as mTGases, including, but not limited to, bacterial transglutaminases (BTG), such as the enzyme with EC index number EC 2.3.2.13 (protein-glutamine-$\gamma$-glutamyltransferase). In some embodiments, mTGase is derived from *Streptomyces ladaccatus* (TG_SL, SEQ ID NO: 15). In some embodiments, mTGase is derived from *Streptomyces mobara* (TG_SM, SEQ ID NO: 16).

Examples

To facilitate understanding of the inventions in the present application, reference will be made to below examples, and the inventions will be described using specific language. However, it should be understood that these specific examples are not intended to limit the scope of the present application. Any alterations and further modifications to the described examples, as well as any further direct or indirect applications of the present applications, are to be apparent to those skilled in the art.

**Example 1: Construction of Expression Vectors and Preparation of Plasmids for Humanized Antibodies**

The murine antibody 17-F12-G8-A6 was humanized to obtain three humanized antibodies: hu17-H2L1, hu17-H3L1, and hu17-H4L1. The variable region sequences of the heavy and light chains of 17-F12-G8-A6 are as set forth in

SEQ ID NOs: 17 and 18 of the Sequence Listing, respectively. The variable region sequences of the heavy and light chains of hu17-H3L1 are as set forth in SEQ ID NOs: 7 and 8 of the Sequence Listing, respectively. The variable region sequences of the heavy and light chains of hu17-H2L1 are as set forth in SEQ ID NOs: 10 and 8 of the Sequence Listing, respectively. The variable region sequences of the heavy and light chains of hu17-H4L1 are as set forth in SEQ ID NOs: 12 and 8 of the Sequence Listing, respectively. The constant region sequences of the heavy and light chains of hu17-H2L1, hu17-H3L1, and hu17-H4L1 are as set forth in SEQ ID NOs: 13 and 14 of the Sequence Listing, respectively.

Preparation of Plasmids

The nucleic acids of interests were amplified by PCR, and the PCR products were purified by gel electrophoresis. The nucleic acid fragments of interests were recovered using a gel extraction kit (Tiangen Biotech, China). The recovered products were then ligated into the PCDNA3.4 plasmid vector to yield recombinant plasmids, which were then transformed into *E. coli* cells. The transformed cells were plated onto plates containing ampicillin for colony screening. Positive colonies were randomly selected for sequencing. Colonies with correct sequences were expanded and cultured, and plasmids were extracted using an endo-free plasmid extraction kit (Tiangen Biotech, China) and verified by further sequencing.

**Example 2: Expression of Humanized Monoclonal Antibodies**

1) $145\times10^6$ CHO cells were collected and centrifuged to remove the supernatant.
2) About 0.5 mL of electroporation buffer was added to and mixed well with the cells. Then, an appropriate amount of the plasmid was added to achieve a concentration of 500 ng/μL.
3) The above cell-plasmid suspension was mixed thoroughly, and 1 mL was transferred into an electroporation cuvette. The cuvette was placed into the electroporation apparatus for electroporation.
4) After electroporation, the cells in the electroporation cuvette were aliquoted into a shake flask containing 20 mL of culture medium and incubated stationarily for 40 min.
5) After incubation, the cells in the shake flask were cultured at 37°C, 270 rpm, and 8% $CO_2$. After 24 hours, sodium butyrate/double antibiotics were added, and cultivation was continued for 3-7 days. On day 5, samples were taken for ELISA testing to confirm the correct expression of humanized monoclonal antibodies hu17-H2L1, hu17-H3L1, and hu17-H4L1.

**Example 3: Purification of Humanized Monoclonal Antibodies**

1) column equilibration: equilibrating the chromatographic column with 1 × PBS (20 mL) at a flow rate of 1 mL/min in an AKTA purifier;
2) sample loading: loading the sample at a flow rate of 1 mL/min;
3) washing: washing the sample with 1 × PBS (20 mL) at a flow rate of 1 mL/min;
4) elution: eluting with sodium acetate buffer (pH3.4) at a flow rate of 1 mL/min, collecting total 10 tubes with about 500 μL per tube, and measuring absorbance at 280 nm using a NanoDrop instrument;
5) dialysis: dialyzing the high-protein-concentration eluates against 1 × PBS (pH 7.0) using a dialysis bag; and
6) production: obtaining monoclonal antibodies with > 95% purity (verified by SEC-HPLC).

**Example 4: Affinity Assessment of Humanized Monoclonal Antibodies**

1. Affinity determination of humanized antibodies using bio-layer interferometry (BLI)

The Octet RED96e system with HIS capture sensors was used to immobilize different antibody samples at 5 μg/mL. ROR1 antigen was serially diluted, starting at 100 nM with 2-fold dilutions for a total of 7 gradients. The buffer was PBS (pH 7.4) with 0.02% Tween 20 and 0.1% BSA. The steps were set as follows: 1) Baseline for 60s, 2) Loading for 30s (antibody immobilization, threshold set to 0.50nm), 3) Baseline 2 for 120s, 4) Association for 120s, 5) Dissociation for 300s, and 6) Regeneration for 30s (Regeneration Sensor). The experimental results were shown in Table 1.

Table 1: Affinity Determination Results of Murine and Humanized Antibodies using Bio-layer Interferometry

| Name | $K_D$(M) | kon(1/Ms) | kdis(1/s) | Full R^2 |
|---|---|---|---|---|
| 17-F12-G8-A6 | 1.48E-08 | 7.08E+05 | 1.05E-02 | 0.9566 |
| hu17-H2L1 | 2.27E-08 | 8.14E+05 | 1.85E-02 | 0.9509 |
| hu17-H3L1 | 3.24E-08 | 8.59E+05 | 2.79E-02 | 0.9521 |
| hu17-H4L1 | 2.00E-08 | 7.38E+05 | 1.47E-02 | 0.9607 |

It was shown that the humanized antibodies hu17-H2L1, hu17-H3 L1, and hu17-H4L1 exhibited affinity comparable to that of the murine antibody 17-F12-G8-A6.

2. Affinity determination of humanized antibodies using enzyme-linked immunosorbent assay (ELISA)

ROR1 protein (ACROBiosystems, Cat# RO1-H5223) was incubated with the anti-ROR1 humanized antibody samples at different concentrations, and then incubated with an IgG-binding secondary antibody (Goat anti-human IgG (H+L) cross-adsorbed secondary antibody (Thermo)). The absorbances at 450 nm for different concentrations were detected by a microplate reader to analyze the affinity of the samples in binding to ROR1 protein. The experimental results were shown in Table 2

Table 2: Affinity Determination Results of Murine and Humanized Antibodies

| Name | 17-F12-G8-A6 | hu17-H2L1 | hu17-H3L1 | hu17-H4L1 |
|---|---|---|---|---|
| $EC_{50}$(ng/mL) | 1.36 | 1.42 | 1.40 | 1.16 |

It was shown that the anti-ROR1 humanized antibodies hu17-H2L1, hu17-H3L1, and hu17-H4L1 exhibited good affinity for ROR1 protein.

3. Affinity determination of humanized antibodies using fluorescence-activated cell sorting (FACS) assay

In this experiment, FACS was used to evaluate the affinity of the anti-ROR1 humanized antibodies in binding to stably transgenic ROR1-expressing cells MDA-MB-231-ROR1 (Kangyuan Biotech). ROR1-positvie MDA-MB-231-ROR1 cells were collected by trypsin digestion, and plated into 96-well plates with $1\times10^5$ cells per well. Then, serially diluted antibodies (starting at 15μg with 5-fold dilutions for a total of 11 gradients) were added, and the plates were incubated at 37°C for 2 h. After washing with PBS, an IgG-binding secondary antibody (Goat anti-human IgG(H+L) cross-adsorbed secondary antibody) was added, and the plates were incubated at 37°C for 1 h. The fluorescence signals were detected by flow cytometry, and binding curves were fitted using GraphPad Prism software. The experimental results were shown in Table 3 and FIG. 2.

Table 3. Affinity Determination Results of Murine and Humanized Antibodies Determined by FACS

| Name | 17-F12-G8-A6 | hu17-H2L1 | hu17-H3L1 | hu17-H4L1 |
|---|---|---|---|---|
| $EC_{50}$(ng/mL) | 98.8 | 114.2 | 106.7 | 100.6 |

It was shown that the anti-ROR1 humanized antibodies hu17-H2L1, hu17-H3L1, and hu17-H4L1 exhibited affinity for the ROR1-expressing tumor cells MDA-MB-231-ROR1 comparable to that of the murine antibody 17-F12-G8-A6.

4. Fc function determination of humanized antibodies using bio-layer interferometry (BLI)

The affinity of the Fc regions of the antibodies was determined using bio-layer interferometry (BLI). The Octet RED96e system with HISIK capture sensors was used to immobilize FcRn, CD64, CD32a, CD32b, CD16 F176, CD16 V176, and C1q at 5 μg/ml. The samples were serially diluted, starting at 100 nM with 2-fold dilutions for a total of 7 gradients. The buffer was PBS (pH 7.4) with 0.02% Tween 20. The steps were set as follows: 1) Baseline for 60s, 2) Loading for 800s (antibody immobilization, threshold set to 0.50nm), 3) Baseline 2 for 120s, 4) Association for 120s, 5) Dissociation for 300s, and 6) Regeneration for 300s (Regeneration Sensor). Blank buffer was used for baseline correction

(y-axis alignment), with data fitting via Savitzky-Golay in Octet Data Analysis Software. The results were shown in Table 4.

By designing point mutations in the Fc region, the antibody hu17-H4L1-AAAS having L234A, L235A, P329A, and P331S mutations in its Fc region, or the antibody hu17-H4L1-AAKS having L234A, L235A, G236K, and P331S mutations in its Fc region can both reduce CD64-mediated ADCP, C1q-mediated CDC, and CD16-mediated ADCC, without affecting FcRn binding, thereby maintaining antibody half-life.

Table 4. Affinity Determination Results of humanized antibodies and FcγRs

| Name | hu17-H4L1-Fc | | | | hu17-H4L1-AAAS | | | | hul7-H4L1-AAKS | | | |
|---|---|---|---|---|---|---|---|---|---|---|---|---|
| Mutations(EU numbering) | NA | | | | L234A/L235A/P329A/P331S | | | | L234A/L235A/G236K/P331S | | | |
| Fortebio | KD (M) | kon(1/Ms) | kdis(1/s) | Full R^\2 | KD (M) | kon(1/Ms) | kdis(1/s) | Full R^2 | KD (M) | kon(1/Ms) | kdis(1/s) | Full R^2 |
| C1q | 1.26E-07 | 2.08E+06 | 2.63E-01 | 0.9903 | 1.40E-10 | 1.25E+04 | 1.75E-06 | 0.2156 | 5.24E-10 | 1.25E+04 | 6.55E-06 | 0.1873 |
| CD16 F176 | 8.23E-07 | 4.88E+04 | 4.01E-02 | 0.9817 | 9.21E-06 | 1.26E+04 | 1.16E-01 | 0.8439 | 4.64E-07 | 2.00E+03 | 9.28E-04 | 0 |
| CD16 V176 | 2.99E-07 | 1.01E+05 | 3.03E-02 | 0.978 | 1.16E-06 | 1.22E+05 | 1.41E-01 | 0.7135 | 7.38E-08 | 2.00E+03 | 1.48E-04 | 0.6928 |
| CD32a | 9.91E-07 | 4.12E+04 | 4.08E-02 | 0.9625 | 9.94E-06 | 6.89E+03 | 6.84E-02 | 0.8931 | 6.66E-04 | 3.09E+01 | 2.06E-02 | 0.5736 |
| CD32b | 1.36E-06 | 1.52E-07 | 2.96E-02 | 0.9784 | 9.34E-06 | 5.21E-06 | 1.02E-01 | 0.915 | <1.0E-12 | 5.13E-05 | <1.0E-07 | 0.583 |
| CD64 | 6.05E-09 | 2.80E+05 | 1.70E-03 | 0.9991 | 2.71E-10 | 1.00E+05 | 2.71E-05 | 0.398 | 3.25E-04 | 1.08E+01 | 3.51E-03 | 0 |
| FcRn | 3.05E-08 | 9.57E+05 | 5.08E-01 | 0.9833 | 2.70E-08 | 1.36E+06 | 7.16E-01 | 0.9915 | 3.09E-08 | 1.14E+06 | 6.04E-01 | 0.9845 |

**Example** 5: **Thermal Stability Evaluation of Humanized Monoclonal Antibodies**

Differential scanning calorimetry (DSC) is widely used to characterize the thermal stability of advanced structures in biological products. In DSC, the heat capacity change of a sample can be measured and analyzed in real time during a controlled temperature ramp. By programmatically controlling the temperature, the relationship between the power difference (heat flow rate) of the sample and the temperature can be determined. Thermodynamic models are then applied to fit and derive thermal stability parameters of the sample including Tonset (onset temperature), Tm (melting temperature), and ΔH (enthalpy change).

The stability of the humanized anti-ROR1 antibodies was analyzed using a Malvern MicroCal PEAQ-DSC system with following parameters:
scanning start temperature: 20°C; scanning end temperature: 100°C; and scanning rate: 90°C/h.

The molecular thermal stability was analyzed by differential scanning calorimetry (DSC), and the results were shown in Table 5.

Table 5. Thermal stability of Murine and Humanized Antibodies by DSC

| Name | Tonset(°C) | Tm1(°C) | Tm2(°C) |
|---|---|---|---|
| 17-F12-G8-A6 | 61.24 | 73.03 | 78.01 |
| hu17-H2L1 | 60.92 | 67.20 | 84.78 |
| hu17-H3L1 | 60.67 | 66.88 | 83.57 |
| hu17-H4L1 | 59.62 | 67.93 | 75.52 |

It is shown that the humanized antibodies hu17-H2L1, hu17-H3L1, and hu17-H4L1 were not significantly different from murine antibody 17-F12-G8-A6 in terms of stability data (Tonset, Tm1, Tm2).

**Example 6: Preparation of ADC**

1. UC961-vcMMAE

The antibody UC961 was diluted in PBS (pH 7.0) containing 5 mM EDTA to a concentration of 10 mg/mL. 3 molar equivalents of TCEP reducing agent (relative to the antibody's molar amount) were added, and the mixture was incubated at 37°C in a water bath for 1 h. Then, 2.5 molar equivalents of vcMMAE (CAS No.: 646502-53-6) were added, and the reaction was carried out at room temperature for 30 min. Subsequently, 2.5 molar equivalents of N-acetyl-L-cysteine were added, and the reaction was stopped after 30 min. Finally, free small molecules and other impurities were removed by ultrafiltration and buffer exchange to obtain UC961-vcMMAE.

2. Preparation of Hu17-H2L1-4LND1002, Hu17-H3L1-4LND1002, and Hu17-H4L1--4LND1002 samples

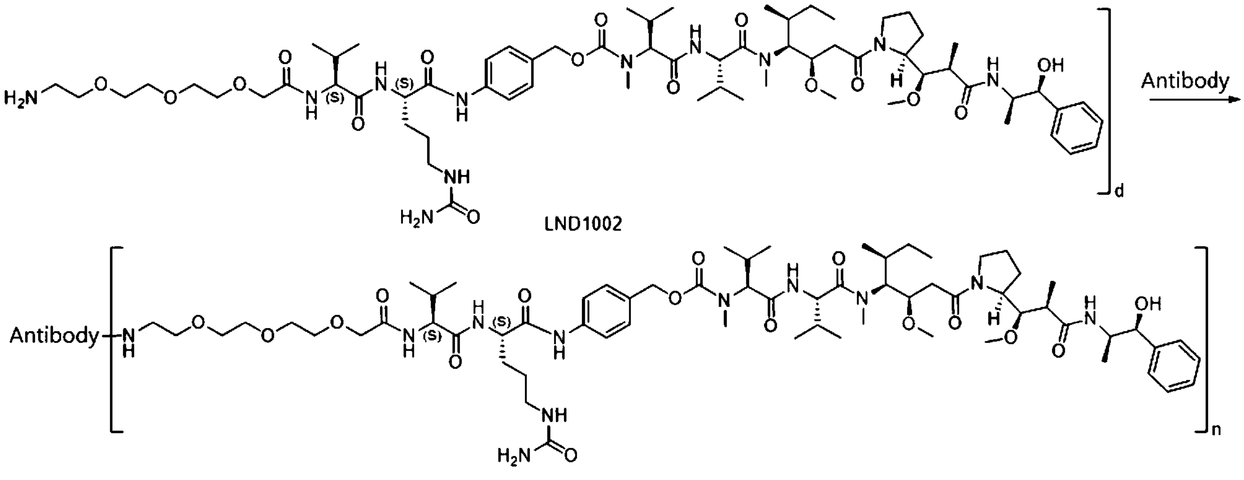

LND1002

A specified volume of LND1002 (containing the drug and the linker $NH_2$-$PEG_3$-Val-Cit-pABC as shown in Fig. 1), the antibody (Hu17-H2L1, Hu17-H3L1 or Hu17-H4L1), mTGase (sequence as set forth in SEQ ID NO: 15, this enzyme was used in all subsequent Examples), and $H_2O$ were added to an EP tube. The tubes were sealed, and the contents were mixed, and incubated at room temperature for no more than 4 days. The reaction was catalyzed by mTGase, forming stable amide bonds (isopeptide bonds) between LND1002 and glutamine residues on the antibodies. The conjugation reaction was considered complete when the heavy chain coupling efficiency exceeded 95%, and ADCs were immediately purified.

Using a sterilized AKTA system, the conjugated reaction mixture was loaded onto a sterilized Protein A column (MabSelect Sure) with 30-35 g ADC/L resin and a minimum residence time of 5 minutes to ensure complete ADC binding. The column was washed extensively with binding/wash buffer (1 ×PBS, pH 7.0) to remove mTGase, unreacted LND1002, and any undesired buffer impurities before the low-pH elution to yield desired products. The eluted fractions were collected in tubes prefilled with a neutralization buffer, yielding antibody-drug conjugates (ADCs) Hul7-H2L1-4LND1002, Hu17-H3L1-4LND1002, and Hu17-H4L1-4LND1002.

**Example 7: Determination of Conjugation Sites in ADCs**

The ADC samples were digested with protease and the digested peptide fragments were analyzed using an ultra-high performance liquid chromatograph and high-resolution mass spectrometer. The raw data were processed using the UNIFI software in UPLC-MS/MS to identify the modification sites in the samples and determine modification intensities. 100 μg of ADC sample was added into a guanidine hydrochloride solution to a final concentration of 6 M, followed by addition of 1 M dithiothreitol (DTT) to a final concentration of 20 mM. The mixture was incubated at 37 °C for 90 min, and cooled to room temperature. Then 1 M iodoacetamide was added to a final concentration of 50 mM. After thorough mixing, the reaction proceeded at room temperature in the dark for 45 min. The reactant was transferred to a 10kDa ultrafiltration membrane, washed with 2M urea (Tris-HCl, pH 7.5), and centrifuged at 13,000 rpm for 10 min to discard the waste liquid. This buffer exchange procedure was repeated twice. Trypsin was then added at enzyme: protein ratio of 1:25 (w:w). After thorough mixing, the reactant was incubated at 37°C for 4 hours, and then, 0.5 μL of FA was added to the digestion solution to terminate the enzymatic digestion. The supernatant was collected by centrifugation for analysis.

The ultra-high performance liquid chromatograph analysis was performed on ACQUITY UPLC H-Class PLUS system (Waters) under the following conditions:

column temperature: 50°C; flow rate: 0.3 mL/min; detection wavelength: 214 nm; mobile phase A: 0.1% formic acid in water; and mobile phase B: 0.1% formic acid in acetonitrile.

The high-resolution mass spectrometry was performed on Vion Q-TOF (Waters) under the following conditions: MSE acquisition mode; capillary voltage (KV): 3V; MSE collision energy: 20-45 eV; and ion source temperature: 120°C.

The raw data were processed using the UNIFI software.

The modification site percentage was calculated as follows:

$$\text{Percent } Q(X) = \text{Area } Q(X)/(\text{Area}(X)+\text{Area } Q(X)) \times 100\%,$$

where Percent Q(X) is the percentage of modified amino acid site X.

The results were shown in Table 6.

Table 6 modification rate of ADC conjugation site

| Name | HC: Q295 | LC: C terminal GGLQSGA |
|---|---|---|
| Hu17-H2L1-4LND002 | 100% | 100% |
| Hu17-H3L1-4LND002 | 100% | 100% |
| Hu17-H4L1-4LND002 | 100% | 100% |

It was shown that the antibody-drug conjugates formed by enzymatic conjugation of humanized antibodies hu17-H2L1, hu17-H3L1, and hu17-H4L1 with LND1002 achieved 100% modification efficiency at Q295 (EU numbering scheme) of the antibody heavy chain (HC) and GGLQSGA at the C-terminal of the antibody light chain (LC).

**Example 8: Determination of Drug-Antibody Ratio (DAR) of ADCs**

1. UC961-vcMMAE(VLS101)

The drug-to-antibody ratio (DAR) distribution was determined by hydrophobic interaction chromatography (HIC-HPLC), and the DAR value was calculated.
Experimental Instruments: High Performance Liquid Chromatography (e2695, Waters) with column: TSKgel Butyl-NPR, 4.6 × 100mm (Cat# 0042168, Tosoh).

Experimental Method:

mobile phase A (20 mM PB, pH 7.0, 1.5 M $(NH_4)_2SO_4$ in water), and mobile phase B (20 mM PB, pH 7.0, 25% isopropanol); controlled flow rate: 0.8 mL/min; column temperature: 30°C; detection wavelength: 280 nm; gradient: 0-20 min, mobile phase B from 0 to 100%; 20-25 min, mobile phase B 100%; and 25-30 min, 100% mobile phase A.
DAR0 is defined as no small molecule conjugated, DAR2 as having two small molecules conjugated, DAR4 as having four small molecules conjugated, DAR6 as having six small molecules conjugated, and DAR8 as having eight small molecules conjugated. Therefore, elution was performed in order of DAR0, DAR2, DAR4, DAR6, and DAR8. The experimental results were analyzed using the area normalization method, and the DAR was about 3.9 (see FIG. 3).

$$\mathrm{DAR} = \sum_{i=0}^{n=8} \mathrm{DARi} \times \mathrm{i}$$

2. Molecular weight verification of Hu17-H2L1-4LND1002, Hu17-H3L1-4LND1002 and Hu17-H4L1-4LND1002

Molecular weight measurements were done by liquid chromatography-mass spectrometry (LC-MS).

Experimental conditions for liquid chromatography:

Column AdvanceBio SEC 200Å, 1.9um, 2.1×150 mm); mobile phase A: 100mM ammonium formate (pH 7.0); isocratic elution for 12 min.

Experimental conditions for mass spectrometry:

Mass spectrometer (6230TOF, Agilent): mass range: 2000-8000 m/z; Vcap: 3500 V; Dry gas Temp: 300°C; Dry Gas Flow 10 L/min; Sheath Gas Temp: 325°C; Sheath Gas Flow: 10 L/min.
The experimental results were shown in Table 7:

Table 7: DAR Values of Hu17-H2L1, Hu17-H3L1, Hu17-H4L1-4LND1002

| Name | Hu17-H2L1-4LND1002 | Hu17-H3L1-4LND1002 | Hu17-H4L1-4LND1002 |
|---|---|---|---|
| Molecular weight | 155871 | 155966 | 155882 |
| DAR(n) | 4.0 | 4.0 | 4.0 |

It was shown that the antibody-drug conjugates formed by enzymatic conjugation of the humanized antibodies hu17-H2L1, hu17-H3L1, and hu17-H4L1 with LND1002 had a DAR value of approximately 4.0.

**Example 9: Thermal Aggregation Temperature Comparison of Antibodies and ADCs**

Dynamic light scattering (DLS) is an optical technique used to measure the size of molecules in a solution or suspension, as well as to analyze the dynamic behavior and structural changes of complex fluids. DLS has been widely applied in characterizing proteins, carbohydrates, polymers, and nanomaterials.
The particle size distribution of the sample was determined using a Wyatt Dyna Pro Plate Reader III High-Throughput Dynamic and Static Light Scattering Analyzer. Before detection, the sample was slowly mixed to ensure homogeneity. 30 μL of the sample was added into a 384-well plate. During the process, contamination by other particles and introduction of air bubbles should be avoided. The 384-well plate was then placed into the analyzer for detection. The steps were set as follows:
1) creating an experiment; 2) maintaining a 25°C constant temperature during the experiment; 3) setting DLS acquisition

time as 5s; and 4) setting DLS data acquisition repetitions as 10 times.

The results were shown in Table 8.

Table 8: Thermal aggregation temperatures comparison of antibodies and ADCs

| Name | Hu17-H2L1 | Hu17-H2L1-4LND1002 | UC961 | UC961-vcMMAE |
|---|---|---|---|---|
| Tagg(°C) | 78.21 | 76.84 | 74.59 | 54.2 |

It was shown that enzymatic conjugation of the antibody Hu17-H2L1 with four LND1002 molecules to form an ADC resulted in a decrease in the thermal aggregation temperature (Tagg) from 78.21°C to 76.84°C, with a reduction of 1.37°C. In contrast, chemical conjugation of the antibody UC961 with four vcMMAE molecules to form an ADC resulted in a decrease in the Tagg from 74.59°C to 54.2°C, with a reduction of 20.39°C. The enzymatic conjugation method for producing ADCs did not affect the interchain disulfide bonds of the antibody, whereas the chemical conjugation method for producing ADCs disrupted the interchain disulfide bonds of the antibody, which more compromises the thermal stability of the antibody. Consequently, the enzymatically conjugated ADC (DAR4) exhibited superior thermal stability compared to chemically conjugated ADC (DAR4).

**Example 10: Internalization of ADCs**

Stably transgenic ROR1-expressing MDA-MB-231 cells (MDA-MB-231-ROR1, Kangyuan Biotech) were collected, resuspended in DMEM medium, and gently pipetted several times to generate a single-cell suspension. Cell viability and count were assessed in trypan blue assays. The cell density was adjusted to $1\times10^5$ cells/mL. 100 μL of the suspension was plated into each well of a 96-well plate, with $1\times10^4$ cells per well. The ADCs (Hu17-H2L1-4LND1002, Hu17-H3L1-4LND1002, and Hu17-H4L1-4LND1002) were added to the wells at a final concentration of 2 μg/mL. The plates were incubated at 37°C with 5% $CO_2$ for 27 hours. Cells were harvested at time points of 3, 6, 9, and 27 hours, resuspended, and stained with goat anti-human Alexa Fluor 488 with a fluorescent label (Thermo Fisher) to quantify surface-bound ADC by flow cytometry (as described in Example 3). The experimental results were shown in FIG. 4.

It was shown that all ADCs (Hu17-H2L1-4LND1002, Hu17-H3L1-4LND1002, and Hu17-H4L1-4LND1002) subjected to endocytosis in MDA-MB-231-ROR1 cells, and the endocytosis rate of Hu17-H2L1-4LND1002 was superior to that of UC961-vcMMAE.

**Example 11: *In vivo* therapeutic efficacy of ADCs**

*1. In vivo* therapeutic efficacy for H1975 human lung adenocarcinoma xenografts

In this experiment, age-desired female NUNU mice were inoculated with H1975 cells to establish H1975 human lung adenocarcinoma nude mouse xenografts. When tumor volumes reached about 100 $mm^3$, 28 mice with desired tumor growth were selected and divided into the following 4 groups (n=7 per group) with tumor volume balancing:

Group A (vehicle control), 0.9% sodium chloride injection (0.9% INJ NS);
Group B (positive reference), UC961-vcMMAE;
Group C (test), Hu17-H2L1-4LND1002; and
Group D (test), Hu17-H3L1-4LND1002.

Mice in all groups were administered weekly for a total of 2 doses. The first dose (day 0, D0) was 3 mg/kg, and the second dose (day 7, D7) was 4 mg/kg. The mice's body weights were recorded after each administration. The tumor growth was dynamically monitored by measuring tumor diameters at various post-administration time points. The experiment was ended on D20 (day 20), at which point mice were euthanized by $CO_2$ asphyxiation, and tumors were excised and weighed. The experimental results were shown in Table 9 and FIG. 5.

Tumor weight inhibition rate (%) = [(Tumor weight in control group - Tumor weight in experimental group) / Tumor weight in control group] × 100%

Table 9: *In vivo* therapeutic efficacy of ADCs in H1975 human lung adenocarcinoma xenografts

| Groups | Dose | Route | Average tumor volume ($mm^3$) | | Tumor weight (g) | TWI (%) |
|---|---|---|---|---|---|---|
| | | | D0 | D20 | | |
| 0.9% INJ NS | -- | i.v qw | 107.7±22.2 | 1,335.1±285.6 | 1.4188±0.4344 | -- |
| UC961-vcMMAE | 3 mg/kg (D0) 4 mg/kg (D7) | | 111.7±26.9 | 569.5±157.9*** | 0.6956±0.3240** | 51.0 |
| Hul7-H2L1-4LND1002 | | | 108.2±20.6 | 307.8±152.7*** | 0.4858±0.2744** * | 65.8 |
| Hul7-H3L1-4LND1002 | | | 108.4±20.2 | 509.1±189.9*** | 0.6570±0.2272** | 53.7 |
| Note: ***: P < 0.001; **: P < 0.01; *: P < 0.05 | | | | | | |

It was shown that the tumor weight inhibition rates (TWI) were determined to be 51.0% for UC961-vcMMAE (VLS101), 65.8% for Hu17-H2L1-4LND1002, and 53.7% for Hu17-H3L1-4LND1002, respectively. In all drug-administered groups, tumor growth was significantly inhibited compared to the vehicle control group. Hu17-H2L1-4LND1002 exhibited significantly better antitumor efficacy than the positive reference UC961-vcMMAE, while the test ADC Hu17-H3L1-4LND1002 exhibited antitumor efficacy comparable to the positive reference.

*2. In vivo* therapeutic efficacy in HCC1187 human breast cancer xenografts

In this experiment, age-desired female NUNU mice were inoculated with HCC1187 cells to establish HCC1187 human breast cancer nude mouse xenografts. When tumor volumes reached about 100 $mm^3$, 28 mice with desired tumor growth were selected and divided into the following 4 groups (n=7 per group) with tumor volume balancing:

Group A (vehicle control group), 0.9% sodium chloride intravenous injection (0.9% INJ NS);
Group B (positive reference), UC961-vcMMAE;
Group C (experimental), Hu17-H2L1-4LND1002; and
Group D (experimental), Hu17-H3L1-4LND1002.

Mice in all groups were administered at a dose of 5 mg/kg on D0, D8, and D12 for a total of three doses. The mice's body weights were recorded after each administration. The tumor growth was dynamically monitored by measuring tumor diameters at various post-administration time points. The experiment was ended on D14 (day 14), at which point mice were euthanized by $CO_2$ asphyxiation, and tumors were excised and weighed. The experimental results were shown in Table 10 and FIG. 6.

Table 10: *In vivo* therapeutic efficacy of ADCs in HCC1187 human breast cancer xenografts

| Groups | Dose | Route | Average tumor volume ($mm^3$) | | Tumor weight (g) | TWI (%) |
|---|---|---|---|---|---|---|
| | | | D0 | D14 | | |
| 0.9% INJ NS | -- | i.v qw | 99.4±15.2 | 1,015.5±122.6 | 1.4753±0.2926 | -- |
| UC961-vcMMAE | 5mpk (D0, D8, D12) | | 99.4±15.4 | 662.0±210.3** | 0.9129±0.2505** | 38.1 |
| Hu17-H2L1-4LND1002 | | | 99.3±14.4 | 541.7±270.2** | 0.6538±0.2502** * | 55.7 |
| Hu17-H3L1-4LND1002 | | | 99.7±14.1 | 626.4±214.0** | 0.7711±0.1918** * | 47.7 |
| Note: ***: P < 0.001; **: P < 0.01; *: P < 0.05 | | | | | | |

It was shown that the tumor weight inhibition rates (TWI) were determined to be 38.1% for UC961-vcMMAE (VLS101), 55.7% for Hu17-H2L1-4LND1002, and 47.7% for Hu17-H3L1-4LND1002, respectively. In all drug-administered groups, tumor growth was significantly inhibited compared to the vehicle control group. The test ADCs Hu17-H2L1-4LND1002 and Hu17-H3L1-4LND1002 exhibited significantly better antitumor efficacy than the positive reference UC961-vcMMAE.

**Example 12: Safety Evaluation in Cynomolgus Monkeys**

Four age-desired cynomolgus monkeys were selected for this experiment. The positive reference UC961-vcMMAE and the test ADC Hu17-H2L1-4LND1002 were administered intravenously, and toxic reactions were recorded.

Both the test ADC and the control ADC had a DAR of approximately 4. The dosage regimen was shown in Table 11. Intravenous administration was done once every three weeks for a total of three doses. The three doses of the control ADC UC961-vcMMAE were 6 mg/kg, 6 mg/kg, and 6 mg/kg, respectively, while the three doses of the test ADC Hu17-H2L1-4LND1002 were 6 mg/kg, 9 mg/kg, and 9 mg/kg, respectively. After administration, the animals were monitored continuously, with general observation twice daily and detailed observation once daily. The animals were weighed one day before administration and weekly after administration. Hematological and blood biochemical parameters were assessed. The experimental design and results of the preliminary toxicology study were shown in Table 11.

Table 11: Experimental Design and Results of Safety Evaluation in cynomolgus monkeys

| | Group | First dose (D1) | Second dose (D22) | Third dose (D43) |
|---|---|---|---|---|
| Dosage | Group 1: VLS101 ( UC961-vcMMAE, Self-manufactured) | 6 mg/kg | 6 mg/kg | 6 mg/kg |
| | Group 2: Hul7-H2L1-4LND1002 | 6 mg/kg | 9 mg/kg | 9 mg/kg |
| Animal number | 2 per group; dosing frequency, Q3W×3 | | | |
| | **Reference group (UC961-vcMMAE)** | | **Test group (Hu17-H2L1-41ND1002)** | |
| General observation | The hairs on the back were sparse from D8 to D50 | | The hairs on the back were sparse from D35 to D50, and then gradually recovered | |
| Body weight/ food intake | From D6 to D13 after administration, the food intake/-body weight decreased and then gradually recovered | | No abnormal changes were observed | |
| Clinical pathology | **Blood cells:** The first dose resulted in white blood cell count (WBC) and neutrophil count (NEUT) reductions of 69% and 98% in the reference group (6 mpk), and 29% and 56% in the test group (6mpk), respectively. The reduction of blood cells in the test group was less than that in the reference group. Both the reference group (6 mg/kg for the first and second doses) and the test group (9mg/kg for the second and third doses) showed similar reductions in WBC, NEUT, and related red blood cell indicators (red blood cell count (RBC), hemoglobin (HGB), and hematocrit (HCT)). | | | |
| | **Blood biochemical index:** Urea, creatinine (Crea), lactate dehydrogenase (LDH), and thyroid globulin (TG) levels were increased and albumin (Alb) was decreased in the reference group (after first dose of 6mg/kg). Before the second dose, one animal in the reference group still had decreased RBC, HGB, and HCT, while other indexed recovered to certain extents. In the test group (after dose of 9mg/kg), there was a transient increase in LDH and decrease in Alb, which then returned to normal. | | | |

It was shown in the safety evaluation study that the toxicity of the ADC Hu17-H2L1-4LND1002 was significantly lower than that of the positive reference UC961-vcMMAE.

**Example 13: TK Study in Cynomolgus Monkeys**

Cynomolgus monkeys were given a single dose of 6 mg/kg of either the test ADC Hu17-H2L1-4LND1002 (represented as H2L1-LND in the figures) or the positive reference UC961-vcMMAE (represented as VLS in the figures). Blood samples were collected immediately after administration (within 1 min), and at 1 h, 4 h, 8 h, 24 h, 48 h, 72 h, 96 h, 120 h, 168 h, 336 h, and 504 h after administration to monitor ADC and free MMAE levels. The results were shown in Tables 12 and 13, and FIGs 7 and 8.

The concentration of small molecules in plasma was determined by LC-MS/MS using the following method.

Under light-shielding conditions, 50 μL of sample was mixed with 200 μL of internal standard working solution and vortexed at 2,500 rpm for 3 min at room temperature. The mixture was centrifuged at 12,000 rpm for 5 min at 2-8°C. 200 μL of supernatant was collected at room temperature for analysis.

Liquid chromatography conditions:

Mobile phase A: 0.1% formic acid in water;

Mobile phase B: 0.1% formic acid in acetonitrile;

column: Waters Xselect® HSS T3 2.5 μm, 2.1× 50 mm column;

flow rate: 0.5mL/min;

injection volume: 5 μL;

autosampler temperature: 4 ± 5°C;

column temperature: 40 ± 5°C; and

elution gradient settings: 0-0.5min, 85% Mobile phase A; 0.5-0.7min, 85%-45% Mobile phase A; 0.7-1.5min, 45%-5% Mobile phase A; 1.5-1.51min, 5%-85% Mobile phase A; and 1.51-2min, 85% Mobile phase A.

Mass spectrometry conditions:

ion source: Electrospray Ionization (ESI);

ion mode: positive ion mode;

detection mode: multiple reaction monitoring (MRM);

spray voltage (ion spray voltage): 5,500V;

turbo ion spray temperature: 550°C;

curtain gas: 35psi;

collision gas: 9psi;

atomizing gas (Gas1): 60psi;

auxiliary Gas (Gas2): 60psi; and

data acquisition time: 2.0min.

Table 12: Pharmacokinetic Parameters of ADCs

| Pharmacokinetic Parameters (unit) | UC961-vcMMAE | | | H2L1-4LND 1002 | | |
|---|---|---|---|---|---|---|
| | D1-6mg/k g | D22-6mg/kg | D43-6mg/kg | D1-6mg/kg | D22-9mg/kg | D43-9mg/kg |
| Cmax(μg/mL) | 232 | 274 | 194 | 199 | 332 | 289 |
| $AUC_{0-t}$(h*μg/mL) | 10939 | 14387 | 12816 | 11144 | 20442 | 22984 |
| $AUC_{0-\infty}$(h*μg/mL) | 11155 | 14526 | 13214 | 11192 | 20595 | 23134 |
| CL(mL/h/kg) | 0.534 | 0.419 | 0.482 | 0.542 | 0.438 | 0.391 |
| MRTO-t(h) | 103 | 92 | 105 | 93.8 | 104.7 | 102.2 |

(continued)

| Pharmacokinetic Parameters (unit) | UC961-vcMMAE | | | H2L1-4LND 1002 | | |
|---|---|---|---|---|---|---|
| | D1-6mg/k g | D22-6mg/kg | D43-6mg/kg | D1-6mg/kg | D22-9mg/kg | D43-9mg/kg |
| Vss(mL/kg) | 61.3 | 40 | 54.3 | 51.7 | 47.7 | 41 |
| Tmax(h) | 0.333 | 0.333 | 0.333 | 0.333 | 0.6665 | 0.333 |
| t1/2(h) | 92.8 | 77.7 | 90.8 | 67.2 | 70.6 | 64.9 |
| Note: Cmax: peak concentration; $AUC_{0-t}$, $AUC_{0-\infty}$: area under the plasma concentration-time curve; CL: clearance rate; $MRT_{0-t}$: mean residence time; Vss: distribution volume; Tmax: time to peak concentration; and t1/2: half-life. | | | | | | |

Table 13: Pharmacokinetic parameters of MMAE

| Pharmacokinetic Parameters (unit) | UC961-vcMMAE | | | H2L1-4LND1002 | | |
|---|---|---|---|---|---|---|
| | D 1-6mg/kg | D22-6mg/kg | D43-6mg/kg | D1-6mg/kg | D22-9mg/kg | D43-9mg/kg |
| Cmax(pg/mL) | 579 | 361 | 361 | 312 | 520 | 496 |
| $AUC_{0-t}$(h*pg/mL) | 84752 | 60676 | 62435 | 56156 | 97368 | 94945 |
| $AUC_{0-\infty}$(h*pg/mL) | 87233 | 62810 | 63637 | 59377 | 99605 | 97172 |
| CL(mL/h/kg) | 69476 | 95671 | 95481 | 101165 | 90561 | 92686 |
| MRTO-t(h) | 127 | 141 | 132 | 136 | 150 | 142 |
| Vss(mL/kg) | 9774752 | 15279184 | 12542392 | 16692230 | 14639293 | 14323754 |
| Tmax(h) | 60 | 36 | 36 | 72 | 40 | 26 |
| t1/2(h) | 94.8 | 110 | 83.1 | 123 | 96 | 100 |
| Note: Cmax: peak concentration; $AUC_{0-t}$, $AUC_{0-\infty}$: area under the plasma concentration-time curve; CL: clearance rate; $MRT_{0-t}$: mean residence time; Vss: distribution volume; Tmax: time to peak concentration; and t1/2: half-life. | | | | | | |

At the same doses, the ADC exposure levels of H2L1-4LND1002 and UC961-vcMMAE were comparable, with an exposure ratio of approximately 1.0; and the free small molecule exposure level of H2L1-4LND1002 was lower than that of UC961-vcMMAE, with an exposure ratio of only 0.7 relative to UC961-vcMMAE. These results demonstrated that while H2L1-4LND1002 showed ADC exposure comparable to UC961-vcMMAE, its free small molecule content was significantly lower, indicating that H2L1-4LND1002 had an improved safety profile, with no significant accumulation of free small molecules observed after administration.

The above description merely illustrates preferred embodiments, which are provided by way of example only and do not limit the combination of features necessary to practice the inventions of the present application. The headings are not intended to limit the various embodiments of the present application. Terms such as "comprising", "containing" and "including" are not intended to be limiting. Further, unless otherwise indicated, lack of numerical modification includes the plural form. "Or" means "and/or". Unless defined otherwise herein, all technical and scientific terms used herein have the same meaning as commonly understood by one skilled in the art. All publications and patents cited in the present application are incorporated herein by reference. Various modifications and variations of the methods and compositions described herein will be apparent to those skilled in the art without departing from the scope and spirit of the present application. While the present application has been described through specific preferred embodiments, it is to be understood that the claimed application should not be unduly limited to these specific embodiments. Indeed, various variations of the described embodiments for carrying out the present application, which will be apparent to those skilled in the relevant art, are intended to be included within the scope of the appended claims.

Sequences in the present application are as follows.

| **Antibody** | **Sequence information (CDRs are defined according to the kabat numbering)** |
|---|---|
| Humanized antibody Hul7-H3L1 | Heavy chain variable region and CDR seauences |

(continued)

| Antibody | Sequence information (CDRs are defined according to the kabat numbering) |
|---|---|
| | QVTLKESGPTLVKPTQTLTLTCSFSGFSLSTSGMGVAWIRQPPGKALEWLAHIWWDDDKRYNPSLKSRLTITKDTSKNQVVLTMTNMDPVDTATYYCARPQFITTVVAFYWYFDVWGQGTTVTVSS (SEQ ID NO: 7)<br>CDR1: TSGMGVA SEQ ID NO: 1<br>CDR2: HIWWDDDKRYNPSLKS SEQ ID NO: 2<br>CDR3: PQFITTVVAFYWYFDV SEQ ID NO: 3<br>Light chain variable region and CDR sequences<br>DILLTQSPSSLSVSVGDRVTITCRASENIYSNLAWYQQKPGKAPKLLVYAATNLADGVPSRFSGSGSGTQYTLTISSLQPEDFATYYCQHFWGTPWTFGGGTKLEIK (SEQ ID NO: 8)<br>CDR1: RASENIYSNLA SEQ ID NO: 4<br>CDR2: AATNLAD SEQ ID NO: 5<br>CDR3: QHFWGTPWT SEQ ID NO: 6 |
| Humanized antibody Hul7-H2L1 | Heavy chain variable region and CDR sequences<br>EVTLKESGPTLVKPTQTLTLTCSFSGFSLSTSGMGVAWIRQPPGKALEWLAHIWWDDDKRYNPALKSRLTITKDTSKNQVVLTITNVDPVDTATYYCARPQFITTVVAFYWYFDVWGQGTTVTVSS (SEQ ID NO: 10)<br>CDR1: TSGMGVA SEQ ID NO: 1<br>CDR2: HIWWDDDKRYNPALKS SEQ ID NO: 9<br>CDR3: PQFITTVVAFYWYFDV SEQ ID NO: 3<br>Light chain variable region and CDR sequences<br>DILLTQSPSSLSVSVGDRVTITCRASENIYSNLAWYQQKPGKAPKLLVYAATNLADGVPSRFSGSGSGTQYTLTISSLQPEDFATYYCQHFWGTPWTFGGGTKLEIK (SEQ ID NO: 8)<br>CDR1: RASENIYSNLA SEQ ID NO: 4<br>CDR2: AATNLAD SEQ ID NO: 5<br>CDR3: QHFWGTPWT SEQ ID NO: 6 |
| Humanized antibody Hul7-H4L1 | Heavy chain variable region and CDR sequences<br>QVTLKESGPTLVKPTQTLTLTCTFSGFSLSTSGMGVAWIRQPPGKALEWLAHIWWDDDKRYGPSLKSRLTITKDTSKNQVVLTMTNMDPVDTATYYCARPQFITTVVAFYWYFDVWGQGTTVTVSS (SEQ ID NO: 12)<br>CDR1: TSGMGVA SEQ ID NO: 1<br>CDR2: HIWWDDDKRYGPSLKS SEQ ID NO: 11<br>CDR3: PQFITTVVAFYWYFDV SEQ ID NO: 3<br>Light chain variable region and CDR sequences<br>DILLTQSPSSLSVSVGDRVTITCRASENIYSNLAWYQQKPGKAPKLLVYAATNLADGVPSRFSGSGSGTQYTLTISSLQPEDFATYYCQHFWGTPWTFGGGTKLEIK (SEQ ID NO: 8) |
| | CDR1: RASENIYSNLA SEQ ID NO: 4<br>CDR2: AATNLAD SEQ ID NO: 5<br>CDR3: QHFWGTPWT SEQ ID NO: 6 |

(continued)

| Antibody | Sequence information (CDRs are defined according to the kabat numbering) |
|---|---|
| Constant region | Heavy chain constant region<br>ASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKVEPKSCDKTHTC PPCPAPEAAGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALAASI EKTISKAKGQPREPQVYTLPPSRDELTKNQVSLTCLVKGFYPSDIAVEWESNGQP ENNYKTTPPVLDSDGSFFLYSKLTVDKSRWQQGNVFSCSVMHEALHNHYTQKS LSLSPGK (SEQ ID NO: 13)<br>Light chain constant region (attached to a glutamine-containing peptide tag):<br>RTVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGECG GLQSGA (SEQ ID NO: 14)<br>Light chain constant region (not attached to a glutamine-containing peptide tag):<br>RTVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC (SEQ ID NO: 19) |
| mTgase TG_SL | |
| mTgase TG_SM | DSDERVTPPAEPLDRMPDPYRPSYGRAETIVNNYIRKWQQVYSHRDGRKQQMT EEQREWLSYGCVGVTWVNSGQYPTNRLAFAFFDEDKYKNELKNGRPRSGETR AEFEGRVAKDSFDEAKGFQRARDVASVMNKALENAHDEGAYLDNLKKELANG NDALRNEDARSPFYSALRNTPSFKDRNGGNHDPSKMKAVIYSKHFWSGQDRSG SSDKRKYGDPEAFRPDRGTGLVDMSRDRNIPRSPTSPGESFVNFDYGWFGAQTE ADADKTVWTHGNHYHAPNGSLGAMHVYESKFRNWSDGYSDFDRGAYVVTFV PKSWNTAPDKVTQGWP (SEQ ID NO: 15)<br>DSDDRVTPPAEPLDRMPDPYRPSYGRAETVVNNYIRKWQQVYSHRDGRKQQM TEEQREWLSYGCVGVTWVNSGQYPTNRLAFASFDEDRFKNELKNGRPRSGETR AEFEGRVAKESFDEEKGFQRAREVASVMNRALENAHDESAYLDNLKKELANGN DALRNEDARSPFYSALRNTPSFKERNGGNHDPSRMKAVIYSKHFWSGQDRSSS ADKRKYGDPDAFRPAPGTGLVDMSRDRNIPRSPTSPGEGFVNFDYGWFGAQTE ADADKTVWTHGNHYHAPNGSLGAMHVYESKFRNWSEGYSDFDRGAYVITFIP KSWNTAPDKVKQGWP (SEQ ID NO: 16) |
| 17-F12-G8-A6 | Heavy chain variable region<br>EVTLKESGPGILQPSQTLSLTCSFSGFSLSTSGMGVAWIRQPSGKGLEWLAHIWW DDDKRYNPALKSRLTISKDTSSNQVFLKIASVDTADTATYYCARPQFITTVVAFY WYFDVWGAGTTVTVSS (SEQ ID NO: 17)<br>Light chain variable region<br>DILLTQSPASLSVSMGETVTITCRASENIYSNLAWYQQKQGKSPQLLVYAATNLA DGVPSRFSGSGSGTQYSLKINSLQSEDFGSYYCQHFWGTPWTFGGGTKLEIK (SEQ ID NO: 18) |

| Antibody | Sequence information (CDRs are defined according to the IMGT numbering) |
|---|---|
| Humanized antibody | Heavy chain variable region CDR sequences<br>CDR1: GFSLSTSGMG SEQ ID NO: 20<br>CDR2: IWWDDDK SEQ ID NO: 21<br>CDR3: ARPQFITTVVAFYWYFDV SEQ ID NO: 22 |

(continued)

| Antibody | Sequence information (CDRs are defined according to the IMGT numbering) |
|---|---|
| Humanized antibody | Light chain variable region CDR sequences<br>CDR1: ENIYSN SEQ ID NO: 23<br>CDR2: AAT<br>CDR3: QHFWGTPWT SEQ ID NO: 6 |

| Antibody | Sequence information (CDRs are defined according to the Chothia numbering) |
|---|---|
| Humanized antibody | Heavy chain variable region CDR sequences<br>CDR1: GFSLSTSGM SEQ ID NO: 24<br>CDR2: WWDDD SEQ ID NO: 25<br>CDR3: PQFITTVVAFYWYFDV SEQ ID NO: 3 |
| Humanized antibody | Light chain variable region CDR sequences<br>CDR1: RASENIYSNLA SEQ ID NO: 4<br>CDR2: AATNLAD SEQ ID NO: 5<br>CDR3: QHFWGTPWT SEQ ID NO: 6 |

| Antibody | Sequence information (CDRs are defined according to the Contact numbering) |
|---|---|
| Humanized antibody | Heavy chain variable region CDR sequences<br>CDR1: STSGMGVA SEQ ID NO: 26<br>CDR2: WLAHIWWDDDKR SEQ ID NO: 27<br>CDR3: ARPQFITTVVAFYWYFD SEQ ID NO: 28 |
| Humanized antibody | Light chain variable region CDR sequences<br>CDR1: YSNLAWY SEQ ID NO: 29<br>CDR2: LLVYAATNLA SEQ ID NO: 30<br>CDR3: QHFWGTPW SEQ ID NO: 31 |

The use of any and all embodiments, or exemplary expressions (e.g. "such as") provided herein, is intended merely to better illuminate the invention and does not pose a limitation on the scope of the invention unless otherwise indicated. No expressions in the specification should be construed as indicating any non-recited element as essential to the practice of the invention.

All publications and patent applications cited in the specification are herein incorporated by reference as if each individual publication or patent application were specifically and individually indicated to be incorporated by reference. Furthermore, any theory, mechanism, attestation, or discovery described herein is intended to further enhance the understanding of the invention and is not intended to limit the invention to such theory, mechanism, proof, or discovery in any way. While the invention has been shown and described in detail in the drawings and foregoing description, the invention is to be considered as illustrative and not restrictive.

## Claims

**1.** An anti-ROR1 antibody or an antigen-binding fragment thereof, comprising a heavy chain variable region and a light chain variable region, wherein:

1) the heavy chain variable region comprises CDR1, CDR2 and CDR3 having the sequences as set forth in SEQ ID NOs: 1, 2 and 3, respectively, and the light chain variable region comprises CDR1, CDR2 and CDR3 having the sequences as set forth in SEQ ID NOs: 4, 5 and 6, respectively; or
2) the heavy chain variable region comprises CDR1, CDR2 and CDR3 having the sequences as set forth in SEQ ID NOs: 1, 9 and 3, respectively, and the light chain variable region comprises CDR1, CDR2 and CDR3 having the sequences as set forth in SEQ ID NOs: 4, 5 and 6, respectively; or
3) the heavy chain variable region comprises CDR1, CDR2 and CDR3 having the sequences as set forth in SEQ

ID NOs: 1, 11 and 3, respectively, and the light chain variable region comprises CDR1, CDR2 and CDR3 having the sequences as set forth in SEQ ID NOs: 4, 5 and 6, respectively.

2. The antibody or antigen-binding fragment of claim 1, wherein the antibody is a monoclonal antibody, a bispecific antibody, or a humanized antibody.

3. The antibody or antigen-binding fragment of claim 1 or 2, wherein the antibody is an IgG-type antibody, preferably an IgG1-type antibody.

4. The antibody or antigen-binding fragment of any one of claims 1 to 3, wherein the antigen-binding fragment is a Fab fragment, a $F(ab')_2$ fragment, or a single-chain Fv fragment (scFv).

5. The antibody or antigen-binding fragment of any one of claims 1 to 4, wherein the heavy chain variable region has the amino acid sequence as set forth in SEQ ID NOs: 7, 10 or 12, and/or the light chain variable region has the amino acid sequence as set forth in SEQ ID NO: 8.

6. The antibody or antigen-binding fragment of any one of claims 1 to 5, wherein the antibody comprises a heavy chain comprising a heavy chain constant region having the amino acid sequence as set forth in SEQ ID NO: 13, and/or the antibody comprises a light chain comprising a light chain constant region having the amino acid sequence as set forth in SEQ ID NO: 19.

7. The antibody or antigen-binding fragment of any one of claims 1 to 6, wherein the antibody or antigen-binding fragment comprises a light chain having a C-terminus attached to a glutamine-containing peptide tag selected from the group consisting of LQSGA, GGLQSGA, and GGGLQSGA.

8. A conjugate comprising the anti-ROR1 antibody or antigen-binding fragment of any one of claims 1 to 7 conjugated to one or more drug molecules, wherein the conjugate has the structure represented by Ab-(L-U)n, where Ab represents the anti-ROR1 antibody or antigen-binding fragment, L represents a linker, U represents the drug molecule, and n is an integer or decimal within the range of 1 to 8, preferably 4.

9. The conjugate of claim 8, wherein the drug molecule is an anticancer drug, such as a cytotoxic drug, an immunopotentiator, or a radioisotope.

10. The conjugate of claim 9, wherein the cytotoxic drug is selected from the group consisting of a tubulin inhibitor, a DNA topoisomerase inhibitor, a DNA damaging agent, an anti-metabolite, and an anti-tumor antibiotic.

11. The conjugate of claim 10, wherein:

the tubulin inhibitor is selected from the group consisting of an auristatin derivative (e.g., MMAE (Monomethyl auristatin E) or MMAF (Monomethyl auristatin F)), and a maytansine alkaloid derivative (e.g., DM1, DM4, Ansamitocin, Mertansine, or Dolastatin or a derivative thereof);
the DNA topoisomerase inhibitor is selected from the group consisting of a camptothecin analogue, and a DNA topoisomerase I inhibitor or a derivative thereof, for example, DXD, SN38, irinotecan, irinotecan hydrochloride, camptothecin, 9-aminocamptothecin, 9-nitrocamptothecin, 10-hydroxycamptothecin, 9-chloro-10-hydroxycamptothecin, 22-hydroxyacuminatine, topotecan, lertonotecan, belotecan, ixitecan, homosilatecan, 6,8-dibromo-2-methyl-3-[2-(D-xylopyranosylamino)phenyl]-4(3H)-quinazolinone, 2-cyano-3-(3,4-dihydroxyphenyl)-N-(phenylmethyl)-(2E)-2-acrylamide, 2-cyano-3-(3,4-dihydroxyphenyl)-N-(3-hydroxyphenylpropyl)-(E)-2-acrylamide, 12-β-D-glucopyranosyl-12,13-dihydro-2,10-dihydroxy-6-[[2-hydroxy-1-(hydroxymethyl)ethyl]amino]-5H-indoleindolo[2,3-a]pyrrolo[3,4-c]carbazole-5,7(6H)-dione, N-[2-(dimethylamino)ethyl]-4-acridine formamide dihydrochloride, or N-[2-(dimethylamino)ethyl]-4-acridinecarboxamide;
the DNA damaging agent is selected from the group consisting of calicheamicin, duocarmycin, and an anthramycin derivative PBD (pyrrolobenzodiazepine);
the anti-metabolite is selected from the group consisting of methotrexate, 6-mercaptopurine, and 5-fluorouracil; and/or
the anti-tumor antibiotic is selected from the group consisting of a polypeptide antibiotic (e.g., actinomycin D or bleomycin) and an anthraquinone (e.g., doxorubicin or mitoxantrone hydrochloride).

12. The conjugate of claim 9, wherein the immunopotentiator is selected from the group consisting of levamisole,

pidotimod, imiquimod, isoprinosine, polyinosinic:polycytidylic acid and polyinosinic:polyuridinic acid.

**13.** The conjugate of claim 9, wherein the radioisotope is selected from the group consisting of $^{211}At$, $^{131}I$, $^{125}I$, $^{90}Y$, $^{186}Re$, $^{188}Re$, $^{153}Sm$, $^{212}Bi$, $^{32}P$, $^{60}Co$, and $^{177}Lu$.

**14.** The conjugate of any one of claims 8 to 13, wherein the drug molecule is conjugated to the antibody or antigen-binding fragment via a linker, and the linker is linked to the antibody or antigen-binding fragment via a sulfhydryl or an amino group.

**15.** The conjugate of claim 14, wherein the linker is independently selected from the group consisting of mc-Val-Cit-pAB, mc-Val-Cit-pABC, mc-Val-Cit, $NH_2$-$(PEG)_m$-Val-Cit, $NH_2$-$(PEG)_m$-Val-Cit-pAB, and $NH_2$-$(PEG)_m$-Val-Cit-pABC, where the m in $(PEG)_m$ is an integer within the range of 1 to 8, preferably 3.

**16.** The conjugate of any one of claims 8 to 15, wherein the antibody comprises a heavy chain constant region comprising Q295, and the linker is linked to the side chain of Q295 via an amide bond; preferably, the linker is linked to the side chain of the glutamine residue from the glutamine-containing peptide tag via an amide bond.

**17.** A pharmaceutical composition comprising the conjugate of any one of claims 8 to 16, a pharmaceutically acceptable carrier, and an antiproliferative agent.

**18.** The pharmaceutical composition of claim 17, wherein the antiproliferative agent is selected from the group consisting of paclitaxel, doxorubicin, docetaxel, cisplatin, carboplatin, and iproplatin.

**19.** Use of the conjugate of any one of claims 8 to 16, or the pharmaceutical composition of claim 17 or 18 in the manufacture of a medicament for the treatment or prevention of cancer.

**20.** A method for treating cancer in a subject, comprising administering to the subject suffering from the cancer a therapeutically effective amount of the conjugate of any one of claims 8 to 16, or the pharmaceutical composition of claim 17 or 18.

**21.** The use of claim 19 or the method of claim 20, wherein the cancer is ROR1-positive cancer.

**22.** The use or method of claim 21, wherein the cancer is a solid tumor selected from the group consisting of gastric cancer, esophageal cancer, pancreatic cancer, lung cancer, ovarian cancer, colon cancer, liver cancer, head and neck cancer, and gallbladder cancer; preferably, an adenocarcinoma of stomach, esophagus, pancreatic duct, bile duct, lung, or ovary; and more preferably, gastric cancer or pancreatic cancer.

**23.** The use or method of claim 21, wherein the cancer is a hematologic tumor, preferably, the hematologic tumor is selected from the group consisting of lymphoma, mantle cell lymphoma, chronic lymphocytic leukemia, diffuse large B-cell lymphoma, follicular lymphoma, non-Hodgkin’s lymphoma, Richter-transformed non-Hodgkin’s lymphoma, T-cell leukemia, Burkitt’s lymphoma, multiple myeloma, marginal zone lymphoma, small lymphocytic lymphoma, marginal zone lymphoma, and marginal cell B-cell lymphoma.

**24.** A medical preparation comprising the conjugate of any one of claims 8 to 16, or the pharmaceutical composition of claim 17 or 18.

**25.** The medical preparation of claim 24, which is a kit comprising a container containing the conjugate or the pharmaceutical composition.

FIG. 1

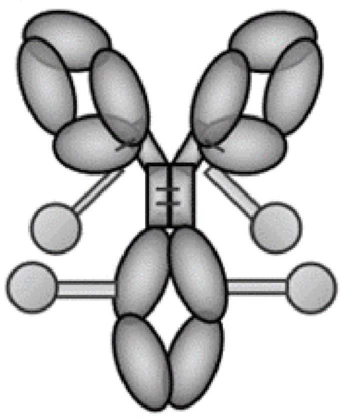

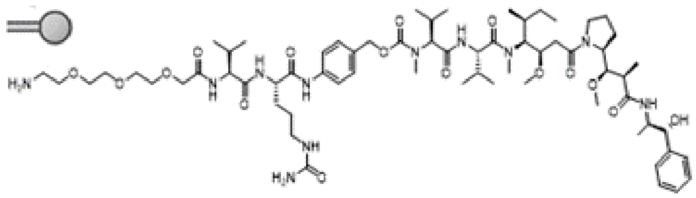

FIG. 2

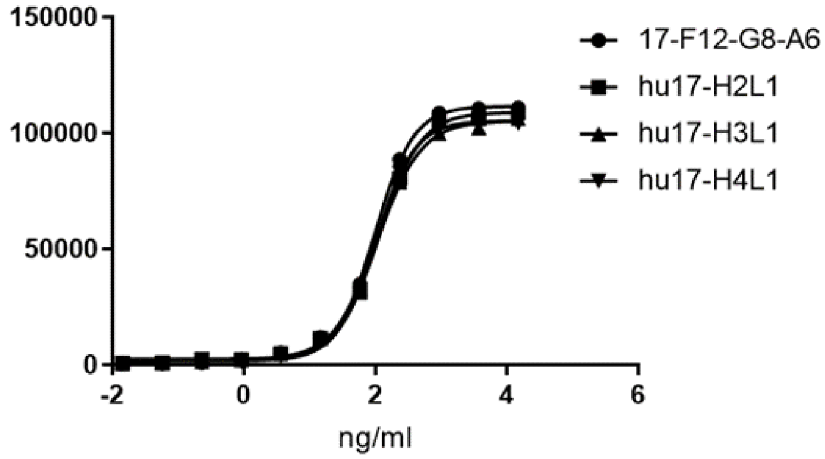


FIG. 3

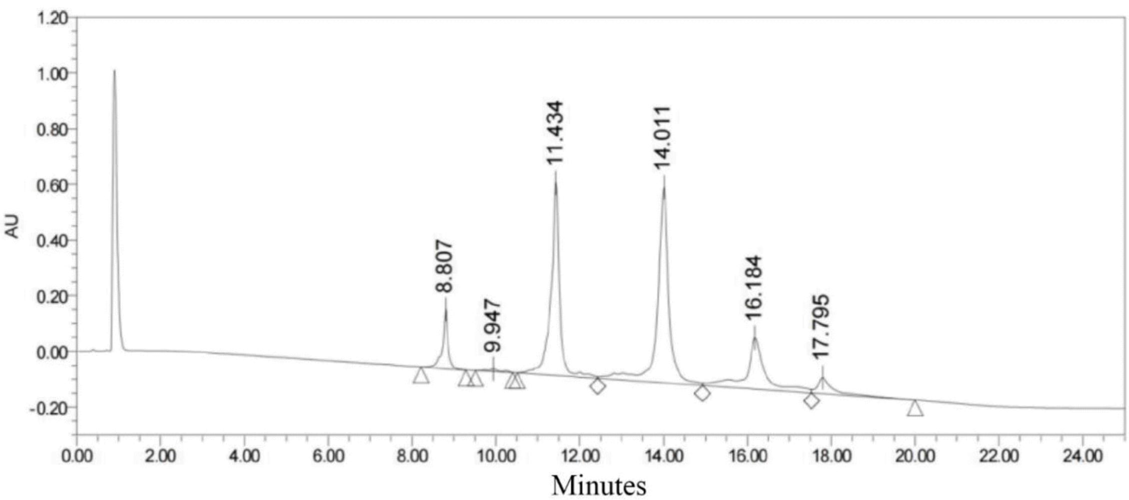

FIG. 4

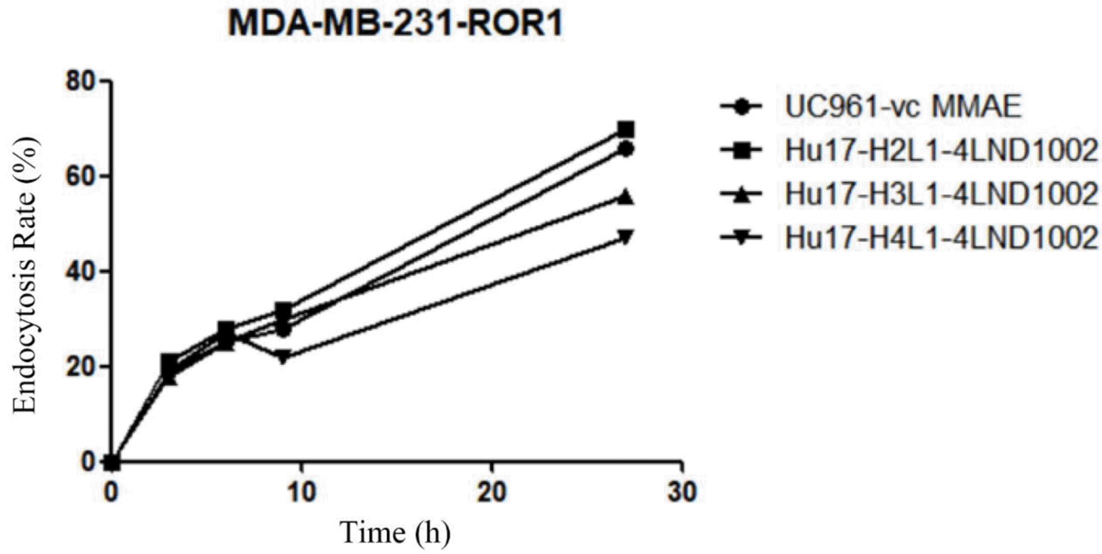
MDA-MB-231-ROR1
80
60
40
20
0
Endocytosis Rate (%)
0
10
20
30
Time (h)
UC961-vc MMAE
Hu17-H2L1-4LND1002
Hu17-H3L1-4LND1002
Hu17-H4L1-4LND1002

FIG. 5

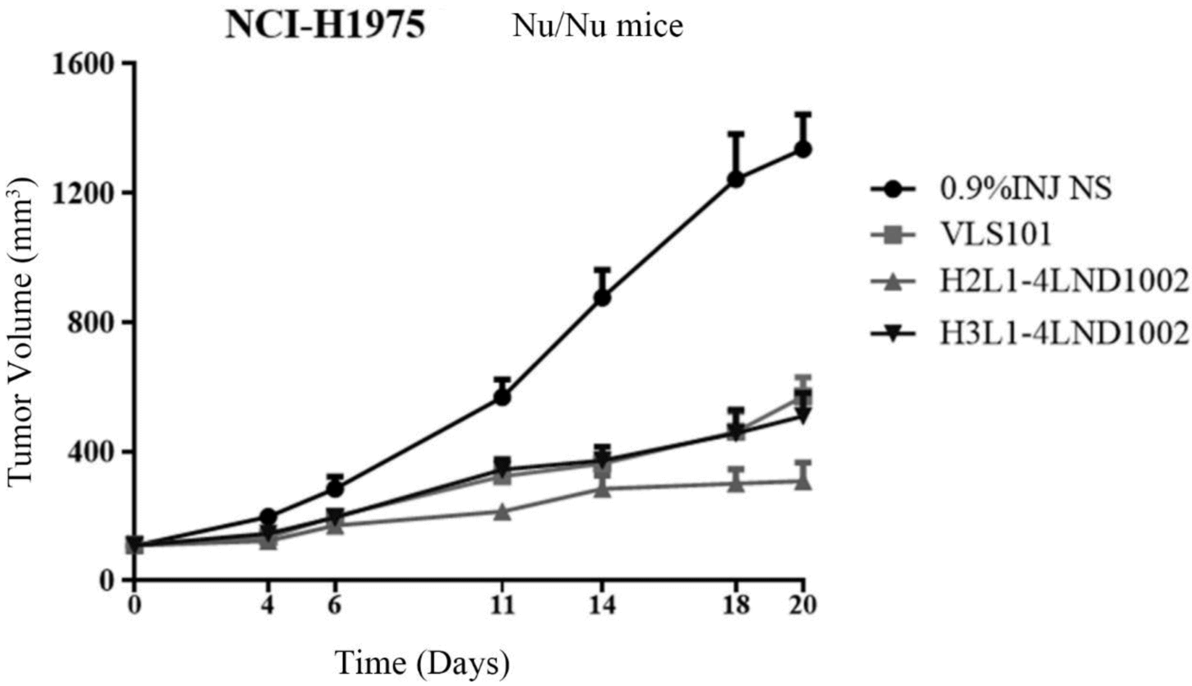
NCI-H1975
Nu/Nu mice
1600
1200
800
400
0
Tumor Volume (mm3)
0
4
6
11
14
18
20
Time (Days)
0.9%INJ NS
VLS101
H2L1-4LND1002
H3L1-4LND1002

FIG. 6

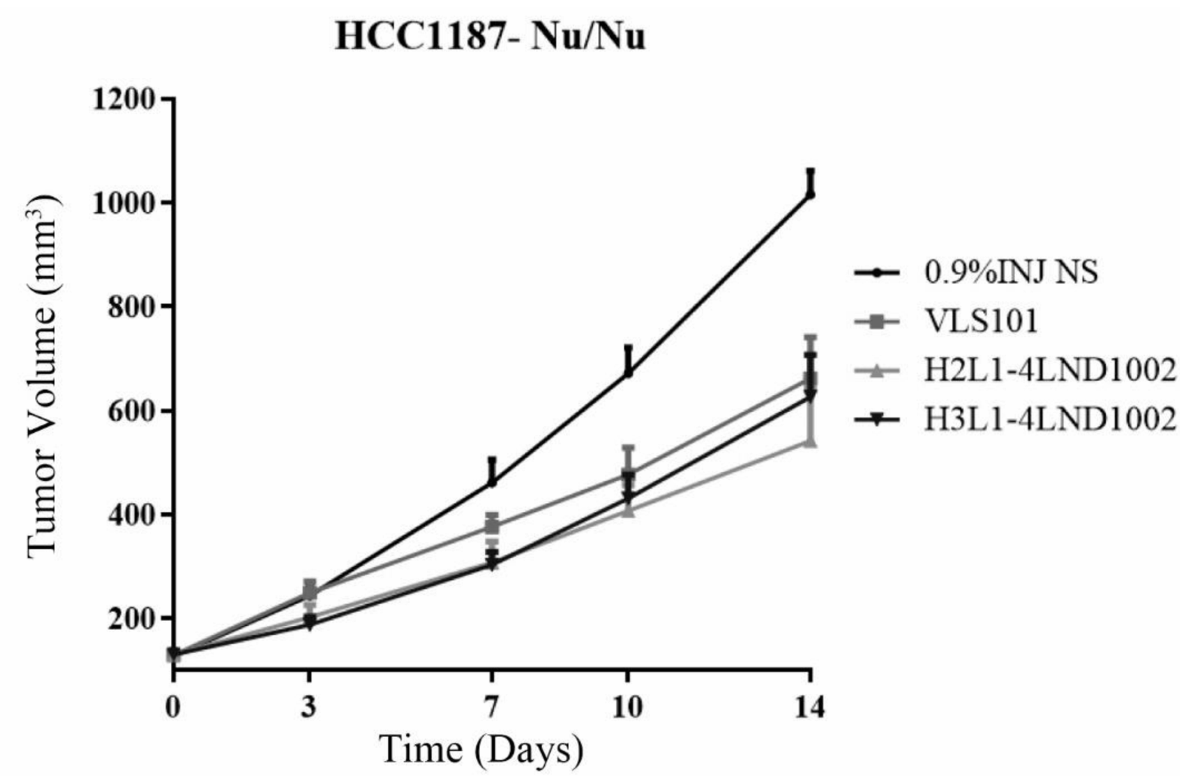
HCC1187- Nu/Nu
Tumor Volume (mm³)
1200
1000
800
600
400
200
0
3
7
10
14
Time (Days)
0.9%INJ NS
VLS101
H2L1-4LND1002
H3L1-4LND1002

FIG. 7

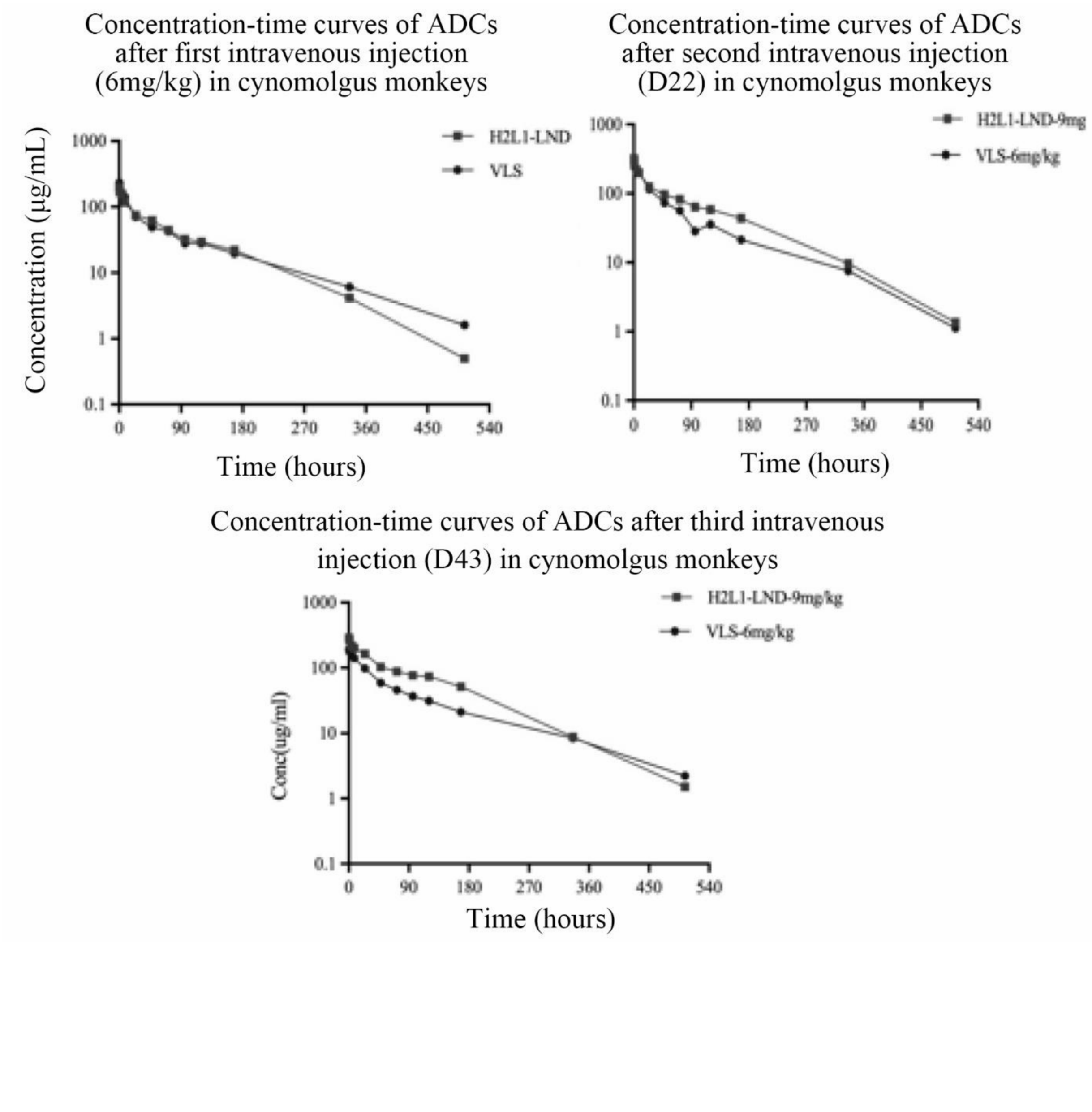
Concentration-time curves of ADCs after first intravenous injection (6mg/kg) in cynomolgus monkeys
Concentration (μg/mL)
1000
100
10
1
0.1
0
90
180
270
360
450
540
Time (hours)
H2L1-LND
VLS
Concentration-time curves of ADCs after second intravenous injection (D22) in cynomolgus monkeys
H2L1-LND-9mg
VLS-6mg/kg
Time (hours)
Concentration-time curves of ADCs after third intravenous injection (D43) in cynomolgus monkeys
Conc(ug/ml)
H2L1-LND-9mg/kg
VLS-6mg/kg
Time (hours)

FIG. 8

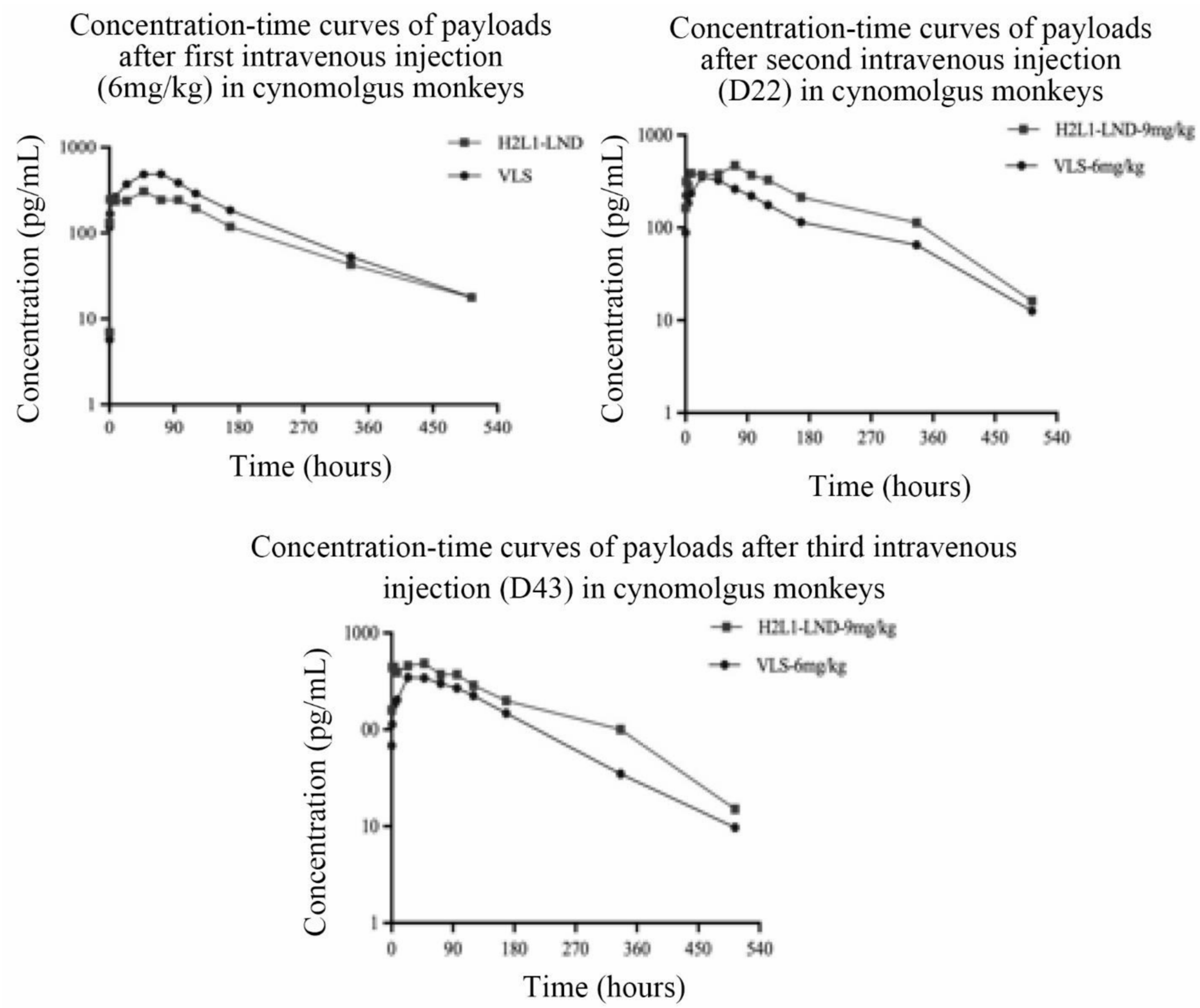
Concentration-time curves of payloads after first intravenous injection (6mg/kg) in cynomolgus monkeys
H2L1-LND
VLS
Concentration (pg/mL)
Time (hours)
Concentration-time curves of payloads after second intravenous injection (D22) in cynomolgus monkeys
H2L1-LND-9mg/kg
VLS-6mg/kg
Concentration (pg/mL)
Time (hours)
Concentration-time curves of payloads after third intravenous injection (D43) in cynomolgus monkeys
H2L1-LND-9mg/kg
VLS-6mg/kg
Concentration (pg/mL)
Time (hours)

**INTERNATIONAL SEARCH REPORT**

International application No.
**PCT/CN2024/108742**

**A. CLASSIFICATION OF SUBJECT MATTER**

C07K16/28(2006.01)i; A61K47/68(2017.01)i; A61P35/00(2006.01)i

According to International Patent Classification (IPC) or to both national classification and IPC

**B. FIELDS SEARCHED**

Minimum documentation searched (classification system followed by classification symbols)

IPC:C07K,A61K,A61P

Documentation searched other than minimum documentation to the extent that such documents are included in the fields searched

Electronic data base consulted during the international search (name of data base and, where practicable, search terms used)

CNTXT, WPABSC, WPABS, ENTXTC, ENTXT, CNKI, Web of Science, google scholar, incoPat, NCBI, EBI, STNext, 万方, WANFANG: ROR1, 抗体, 人源化, 癌症, antibod+, humanized antibod+, SEQ ID NOs: 1-5, 9 and 11

**C. DOCUMENTS CONSIDERED TO BE RELEVANT**

| Category* | Citation of document, with indication, where appropriate, of the relevant passages | Relevant to claim No. |
|---|---|---|
| A | CN 111587124 A (VELOSBIO INC.) 25 August 2020 (2020-08-25)<br>abstract, and claims 1-41 | 1-25 |
| A | CN 110590951 A (ANMENGDE MEDICAL TECHNOLOGY (SHANGHAI) CO., LTD.) 20 December 2019 (2019-12-20)<br>abstract, and claims 1-10 | 1-25 |
| A | CN 113521300 A (CSTONE PHARMACEUTICALS (SUZHOU) CO., LTD. et al.) 22 October 2021 (2021-10-22)<br>abstract, and claims 1-16 | 1-25 |
| A | CN 114539411 A (SHANDONG BOAN BIOTECHNOLOGY CO., LTD.) 27 May 2022 (2022-05-27)<br>abstract, and claims 1-13 | 1-25 |
| A | WO 2021044208 A1 (LEGOCHEM BIOSCIENCES, INC. et al.) 11 March 2021 (2021-03-11)<br>abstract, and claims 1-130 | 1-25 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **04 November 2024** | **11 November 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/108742**

**C. DOCUMENTS CONSIDERED TO BE RELEVANT**

| Category* | Citation of document, with indication, where appropriate, of the relevant passages | Relevant to claim No. |
|---|---|---|
| A | VAISITTI, T. et al. "ROR1 Targeting with the Antibody-Drug Conjugate VLS-101 is Effective in Richter Syndrome Patient-Derived Xenograft Mouse Models" *Blood*, Vol. 137, No. (24), 17 June 2021 (2021-06-17), abstract, and page 3374 | 1-25 |

Form PCT/ISA/210 (second sheet) (July 2022)

**INTERNATIONAL SEARCH REPORT**

International application No.
**PCT/CN2024/108742**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/108742**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **20, 21-23 (in part)**
because they relate to subject matter not required to be searched by this Authority, namely:

Claims 20 and 21-23 (in part) relate to a method for treating a disease, which falls within methods for treatment of diseases as defined in PCT Rule 39.1(iv). However, a search is still performed on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/108742**

| Patent document cited in search report | Publication date (day/month/year) | Patent family member(s) | Publication date (day/month/year) |
|---|---|---|---|
| CN 111587124 A | 25 August 2020 | AU 2018289581 A1 | 16 January 2020 |
| | | AU 2018289581 B2 | 19 September 2024 |
| | | ZA 201908351 B | 28 April 2022 |
| | | MX 2019015057 A | 03 August 2020 |
| | | IL 271575 A | 27 February 2020 |
| | | BR 112019027448 A2 | 07 July 2020 |
| | | JP 2020525542 A | 27 August 2020 |
| | | JP 7245239 B2 | 23 March 2023 |
| | | KR 20200062161 A | 03 June 2020 |
| | | WO 2018237335 A1 | 27 December 2018 |
| | | KR 20210079427 A | 29 June 2021 |
| | | US 2020030454 A1 | 30 January 2020 |
| | | JP 2023036631 A | 14 March 2023 |
| | | JP 7512361 B2 | 08 July 2024 |
| | | EP 3641830 A1 | 29 April 2020 |
| | | MX 2021007620 A | 11 August 2021 |
| | | TW 201919709 A | 01 June 2019 |
| | | TWI 804499 B | 11 June 2023 |
| | | CA 3067829 A1 | 27 December 2018 |
| | | US 2022133901 A1 | 05 May 2022 |
| | | US 2018369406 A1 | 27 December 2018 |
| | | US 10335496 B2 | 02 July 2019 |
| | | AU 2018289581 A1 | 16 January 2020 |
| | | AU 2018289581 B2 | 19 September 2024 |
| | | ZA 201908351 B | 28 April 2022 |
| | | MX 2019015057 A | 03 August 2020 |
| | | IL 271575 A | 27 February 2020 |
| | | BR 112019027448 A2 | 07 July 2020 |
| | | JP 2020525542 A | 27 August 2020 |
| | | JP 7245239 B2 | 23 March 2023 |
| | | KR 20200062161 A | 03 June 2020 |
| | | WO 2018237335 A1 | 27 December 2018 |
| | | KR 20210079427 A | 29 June 2021 |
| | | US 2020030454 A1 | 30 January 2020 |
| | | JP 2023036631 A | 14 March 2023 |
| | | JP 7512361 B2 | 08 July 2024 |
| | | EP 3641830 A1 | 29 April 2020 |
| | | MX 2021007620 A | 11 August 2021 |
| | | TW 201919709 A | 01 June 2019 |
| | | TWI 804499 B | 11 June 2023 |
| | | CA 3067829 A1 | 27 December 2018 |
| | | US 2022133901 A1 | 05 May 2022 |
| | | US 2018369406 A1 | 27 December 2018 |
| | | US 10335496 B2 | 02 July 2019 |
| CN 110590951 A | 20 December 2019 | None | |
| CN 113521300 A | 22 October 2021 | JP 2021063058 A | 22 April 2021 |
| | | IL 314603 A | 01 September 2024 |
| | | CA 3153069 A1 | 11 March 2021 |
| | | EP 4025257 A1 | 13 July 2022 |
| | | EP 4025257 A4 | 22 November 2023 |

Form PCT/ISA/210 (patent family annex) (July 2022)

**INTERNATIONAL SEARCH REPORT**
**Information on patent family members**

International application No.
**PCT/CN2024/108742**

| Patent document cited in search report | Publication date (day/month/year) | Patent family member(s) | Publication date (day/month/year) |
|---|---|---|---|
| | | KR 20210028544 A | 12 March 2021 |
| | | US 2023405139 A1 | 21 December 2023 |
| | | AU 2020343888 A1 | 17 March 2022 |
| | | MX 2022002592 A | 25 March 2022 |
| | | JP 2023179421 A | 19 December 2023 |
| | | WO 2021044208 A1 | 11 March 2021 |
| | | WO 2021044208 A8 | 01 April 2021 |
| | | TW 202122115 A | 16 June 2021 |
| | | TW 202408591 A | 01 March 2024 |
| | | US 2021069342 A1 | 11 March 2021 |
| | | US 11707533 B2 | 25 July 2023 |
| | | AU 2024205092 A1 | 29 August 2024 |
| | | BR 112022003589 A2 | 24 May 2022 |
| | | IL 290979 A | 01 May 2022 |
| CN 114539411 A | 27 May 2022 | None | |
| WO 2021044208 A1 | 11 March 2021 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 111587124 A


### Non-patent literature cited in the description


- **VERHOEYN et al.** *BioEssays*, 1988, vol. 8, 74
- **HARLOW** ; **LANE**. Antibodies: A Laboratory Manual. Cold Spring Harbor Laboratory, 1988, 726
- **KOHLER** ; **MILSTEIN**. *Nature*, 1975, vol. 256, 495-497
- *CHEMICAL ABSTRACTS*, 202350-68-3
- *CHEMICAL ABSTRACTS*, 646502-53-6